# EUROPEAN PATENT APPLICATION

(11) **EP 4 809 918 A1**
(43) Date of publication of application: **23.09.2026**
(21) Application number: 25164187.4
(22) Date of filing: 17.03.2025
(51) Int. Cl.: A61B 5/145, A61B 5/15, A61B 5/00, A61M 5/158

(54) **PATCH-LIKE MEDICAL DEVICE WITH A SENSOR DEVICE**

(71) Applicant: PharmaSens AG, 2503 Biel/Bienne (CH)
(72) Inventor: AUDERSET, Adrian, 2560 Nidau (CH); MESSERLI, Hans, 4566 Kriegstetten (CH); SCHOEMAKER, Michael, 67454 Hassloch (DE); SCHILTGES, Gilbert, 4512 Bellach (CH); SCHRANZ, Samuel, 3252 Worben (CH); HÜGLI, Serge, 3422 Kirchberg (CH); BOTH, Marcel, 3038 Kirchlindach (CH)
(74) Representative: Mayer, Aline Sophie

(57) **Abstract**

A patch-like medical device is disclosed comprising a base (10) having a contact surface (11) configured to be directed towards a patient's skin and a sensor device (15) comprising an insertable portion (151) configured to measure an analyte concentration subcutaneously, the insertable portion (151) being fixedly positioned relative to the base (10) and protruding from the contact surface (11), wherein the patch-like medical device (1) further comprises at least one of: a telescopic sleeve assembly (6) which is collapsible along a central axis (A) from a first state into a second state; a non-retractable insertion support for inserting the insertable portion (151) of the sensor device or a sensor insertion mechanism comprising a retractable sharp (161); a delivery needle (12) fixedly positioned relative to the base (10) having a delivery needle tip (121) which protrudes from the contact surface (11) and which is configured for piercing the patient's skin.

## Description

### TECHNICAL FIELD

The present invention relates to a patch-like medical device configured to be applied to a patient's skin with a sensor device comprising an insertable portion which is configured to measure an analyte concentration subcutaneously.

### PRIOR ART

Monitoring of an analyte concentration in a patient's body can be of crucial importance to determining the patient's health state. Especially for patients dealing with chronical medical conditions, being able to monitor the concentration of certain analytes, in particular glucose in the case of diabetes, is key to being able to prevent a critical situation, e.g. hypoglycemia or hyperglycemia. Patch-like devices with a sensor device having an insertable portion configured to measure an analyte concentration subcutaneously over an extended period of time while the patch-like device is worn on the skin by the patient can allow patients to keep track of their health state while pursuing everyday activities.

However, patients may be reluctant to use such a patch-like device if the device is bulky, if the insertable portion is prone to damage prior to use, if there is a risk of unintentional exposure to a pointed element prior to insertion, if the pointed element is visible for the patient prior to insertion, if proper insertion of the insertable portion of the sensor device is difficult or complicated, e.g. because it may require proper use of an additional inserter device, or if the device cannot be easily combined with subcutaneous administration of a necessary liquid substance, e.g. an insulin formulation.

### SUMMARY OF THE INVENTION

In a first aspect, it is an object of the present invention to provide a patch-like medical device with a sensor device comprising an insertable portion configured to measure an analyte concentration subcutaneously with a protection mechanism for protecting the insertable portion from damage and protecting a user from unintentional pricking prior to use while still allowing the patch-like medical device to be compact and slim to enhance the patient's comfort.

This object is achieved by a patch-like medical device according to claim 1. Further embodiments of the invention are laid down in the dependent claims.

The present invention provides a patch-like medical device comprising:
a base having a contact surface configured to be directed towards a patient's skin;
a sensor device comprising an insertable portion which is configured to measure an analyte concentration subcutaneously, wherein the insertable portion is fixedly positioned relative to the base and protrudes from the contact surface;
a telescopic sleeve assembly which is collapsible along a central axis from a first state into a second state,
wherein in the first state, the telescopic sleeve assembly has a first assembly length in direction of the central axis and extends beyond the insertable portion, and
wherein in the second state, the telescopic sleeve assembly has a second assembly length in direction of the central axis that is shorter than the first assembly length and the insertable portion protrudes from the telescopic sleeve assembly.

The central axis is preferably parallel to or identical with an axis along which the insertable portion of the sensor device extends, and is preferably perpendicular to the contact surface.

In the present context, the term "axial direction" refers to a direction parallel to the central axis. The term "distal" refers to a direction parallel to the central axis leading away from the contact surface, i.e. towards the patient's skin when the patch-like medical device is applied as intended. The term "proximal" refers to a direction which is 180° opposite to the distal direction, i.e. leading away from the patient's skin when the patch-like medical device is applied as intended. According to this definition, the telescopic sleeve assembly extends distally beyond the insertable portion in the first state and collapses in proximal direction from the first state into the second state.

In the present context, the expression "insertable portion which is configured to measure an analyte concentration subcutaneously" is used to describe a sensing portion of the sensor device, i.e. a portion of the sensor device which comprises sensing means to measure the analyte concentration in the patient's subcutaneous tissue. The sensor device may further comprise a non-insertable portion, which is arranged inside the patch-like medical device. As described further below, the sensor device or the patch-like medical device may comprise further components that are not part of the insertable portion of the sensor device according to this definition, but that can also be "insertable" in the sense that they are configured to pierce the patient's skin.

The insertable portion of the sensor device may protrude from the contact surface by an insertion portion length of between 1 and 12 mm, in particular between 4 and 8 mm, preferably between 5 and 7 mm.

Preferably, the insertable portion of the sensor device comprises a set of electrodes, in particular a working electrode, a reference electrode and a counter electrode, which are configured to measure the analyte concentration in an electrochemical manner which is known in the art. In some embodiments, the reference electrode and the counter electrode are physically separate electrodes. In other embodiments, the insertable portion may comprise a first electrode acting as the working electrode and a second electrode acting as both the reference electrode and the working electrode.

Alternatively, the insertable portion may comprise an optical sensor configured to measure the analyte concentration in an optical manner.

The analyte to be measured may in particular be glucose.

The patch-like medical device may comprise electronic circuitry to operate the sensor device. In particular, the electronic circuitry may be configured as a potentiostat for applying a well-defined potential difference between the working electrode and the reference electrode while minimizing a current through the reference electrode and for measuring a current that flows through the working electrode and/or through the counter electrode.

Furthermore, the electronic circuitry may comprise a power source, e.g. a non-rechargeable battery and/or a rechargeable battery and/or an induction coil, one or more processors for controlling the sensor device and/or for data processing, a transmitter and/or receiver for wireless communication to a remote device, a temperature sensor, and/or an A/D-converter. The electronic circuitry may comprise a printed circuit board (PCB) with individual components or may comprise an application-specific integrated circuit (ASIC).

Preferably, the patch-like medical device is configured to be worn for a period of 2 to 20 days, in particular 3 to 7 days.

The patch-like medical device may have a first device part with the base forming a housing lower part, and a second device part with a housing upper part couplable to the base. The coupling may be reversible so that the parts can be separated again after use. Alternatively, the coupling may be irreversible so that it is no longer possible to release the coupling. The coupling may be made by the user prior or after insertion of the insertable portion or at the factory during manufacturing. Preferably, the parts are configured in a way to support a watertight coupling.

In some embodiments, the electronic circuitry to operate the sensor device may be arranged in the second device part. In such a case, the second device part is preferably reusable, while the first device part, which comprises the insertable portion of the sensor device, may be disposable, i.e. meant to be disposed after having been worn on the skin.

Alternatively, the patch-like medical device may be a single-housing device, i.e. without separable parts. In particular, it may be a single-use device meant to be disposed in its entirety after having been worn.

The non-insertable portion of the sensor device may comprise a contact portion with contact pads that are connected to the insertable portion, in particular to the electrodes, via conductive traces. If the patch-like medical device has two parts as described above, the non-insertable portion of the sensor device is preferably arranged in the first device part and comprises a disconnectable interface to the electronic circuitry, which is arranged in the second device part, such that the electronic circuitry may be safely connected and disconnected to the sensor device via said interface when the first device part and the second device part are being separated to dispose of and/or replace the first device part. The disconnectable interface may comprise or be provided by the contact pads.

Having the insertable portion of the sensor device being fixedly positioned relative to the base provides enhanced mechanical stability and facilitates electrical connectability, since there is no need for electrical wiring which has to be flexible or movable with respect to the base. Furthermore, it allows for the analyte concentration to be measured at a pre-defined distance from the contact surface, and thus from the surface of the patient's skin, and therefore does not depend on an insertion depth which may vary if the insertable portion of the sensor device is movable with respect to the base and the contact surface and may therefore inadvertently not always be inserted at the same depth.

Since the second assembly length of the telescopic sleeve assembly, i.e. the length of the telescopic sleeve assembly in axial direction from a proximal end of the assembly to a distal end of the assembly when the telescopic assembly is collapsed, is shorter than the first assembly length, i.e. length of the telescopic sleeve assembly from a proximal end of the assembly to a distal end of the assembly when the telescopic assembly is in its initial extended state, the telescopic sleeve assembly takes up less space when collapsed. The telescopic sleeve assembly thus provides a compact protection mechanism that allows the patch-like medical device to be slimmer in axial direction and thus more comfortable for the patient.

In the second state, the telescopic sleeve assembly is preferably arranged inside the patch-like medical device and does not protrude from the contact surface. In particular, a distal end of the telescopic sleeve assembly may be flush with the contact surface when the telescopic sleeve assembly is in the second state.

In some embodiments, the base may simply have a through-hole, and the telescopic sleeve assembly may be received in a cavity formed between the base and the housing upper part. Preferably however, the base defines a recess on the side that faces away from the contact surface for receiving the telescopic sleeve assembly. The recess may be delimited in axial direction by a top wall. In radial direction, the recess may be delimited by a side wall, the side wall being preferably cylindrical.

The telescopic sleeve assembly may in particular comprise:
a protection sleeve movable along the central axis from a first position in the first state to a second position in the second state, the protection sleeve extending beyond the insertable portion in the first position, and exposing the insertable portion in the second position;
a rotating sleeve movable along the central axis, the rotating sleeve being in a thread-like engagement with the protection sleeve such that a helical movement of the rotating with respect to the base causes the protection sleeve to move along the central axis from the first position to the second position;
a rotation lock sleeve movable along the central axis while being rotationally fixed with respect to the base, the rotation lock sleeve being in engagement with the protection sleeve such that the protection sleeve is prevented from rotating with respect to the base when the protection sleeve moves from the first position to the second position.

The protection sleeve may be at least partially received radially inside the rotating sleeve. The rotating sleeve and the sidewall delimiting the recess defined by the base may be in a thread-like engagement with each other. Preferably, the thread-like engagement of the protection sleeve with the rotating sleeve exhibits a handedness that is opposite to the handedness of the thread-like engagement of the rotating sleeve with the side wall that delimits the recess.

In the present context, a "thread-like engagement" refers to a mechanical engagement between a first part and a second part that causes a screwing motion, i.e. a combined axial and rotational movement, of the first part relative to the second part. In particular, a thread-like engagement may involve a thread, for instance in form of a helical groove, on the first part and a thread or thread portions, for instance in form of helical ribs, on the second part, the thread portions of the second part being configured to engage with said thread of the first part. The threads and the thread portions may in particular be square threads, i.e. have a square profile.

The patch-like medical device may further comprise a fixation element that is fixedly attached to the base and is in engagement with the rotation lock sleeve such that the rotation lock sleeve is prevented from rotating relative to the base, wherein the fixation element is preferably configured as a fixation ring.

The fixation element may comprise a notch, and the rotation lock sleeve may have an outer surface comprising a ridge extending parallel to the movement axis and being in engagement with the notch of the fixation ring. Preferably, the fixation ring comprises two to four notches, ideally equally distributed along a circumference of the fixation ring for better stability, and the rotation lock sleeve accordingly comprises two to four ridges.

Furthermore, the fixation element may act as a stop element against which the rotation lock sleeve may axially abut in distal direction when the protection sleeve is in the first position, thus preventing the rotation lock sleeve and the rotating sleeve from disengaging from the base. The fixation element may be a separate piece attached to the base or may be made in one piece with the base. In case the fixation element is a separate piece, it may be attached to the casing by soldering, screwing or gluing.

In order to further facilitate insertion of the insertable portion of the sensor device, the patch-like medical device may further comprise:
a sensor insertion mechanism comprising a sharp having a sharp tip configured for piercing the patient's skin, the sharp being movable from an insertion position to a retracted position,
wherein in the insertion position, the insertable portion of the sensor device is at least partially received within the sharp and the sharp tip extends beyond the insertable portion of the sensor device to enable insertion of the insertable portion into the patient's skin, and
wherein in the retracted position, the insertable portion of the sensor device is exposed.

In the present context, the term "sharp" refers to any elongated element configured to pierce the patient's skin. In particular, the sharp may have a U-shaped profile or may be a hollow needle. Providing a retractable sharp to insert the insertable portion of the sensor device allows said insertable portion to be mechanically flexible, which may enhance the comfort for the patient if the patch-like medical device is worn over an extended period of time.

To enable an automated retraction and thus further enhance the patient's comfort, the sensor insertion mechanism may comprise a retraction drive mechanism configured to drive the movement of the sharp from the insertion position to the retracted position.

The retraction drive mechanism may comprise a sharp retraction spring for driving the movement of the sharp, wherein in the insertion position, the sharp retraction spring is in a biased state, in particular in a compressed state. In the retraced position, the sharp retraction spring may be in an at least partially relaxed state.

To prevent inadvertent retraction of the sharp prior to insertion of the insertable portion of the sensor device into the patient's skin, the sensor insertion mechanism may further comprise a retraction-lock mechanism having a retraction-lock state, in which the sharp is held in the insertion position, and a released state, in which the sharp is allowed to move to the retracted state. The sensor insertion mechanism may further comprise a retraction trigger mechanism configured to switch the retraction-lock from the retraction-lock state to the released state.

In some embodiments, the retraction trigger mechanism may comprise a switch element to switch the retraction-lock mechanism from the retraction-lock state to the released state. The switch element may comprise or consist of a shape-memory alloy. In particular, the shape-memory alloy may be a metal alloy of nickel and titanium, e.g. nitinol, or a copper (Cu)-based alloy. The switch element may in particular be switched between two different states in which the switch element exhibits a different geometrical shape. The switch element may be switched by heating or cooling the switch element. Heating of the switch element may in particular be effected by applying a DC-current to the switch element, or by heating it with a separate heating element, such as a heating foil or a heating wire.

In some embodiments, the retraction trigger mechanism for retracting the sharp may be configured to cooperate with a different trigger mechanism, in particular a trigger mechanism which is used to trigger the collapse of the telescopic sleeve assembly from its first state to its second state. In such a case, the patch-like medical device may further comprise a locking mechanism having a locked state in which the locking mechanism prevents the telescopic sleeve assembly from collapsing into the second state when the telescopic sleeve assembly is in the first state, and an unlocked state in which the locking mechanism allows the telescopic sleeve assembly to collapse into the second state, and a trigger mechanism configured to switch the locking mechanism from the locked state to the unlocked state, wherein the trigger mechanism for switching the locking mechanism from the locked state to the unlocked state and the retraction trigger mechanism for switching the retraction-lock mechanism from the retraction-lock state to the released state share a movable component.

The movable component may be movable from a sleeve-unlocking position to a retraction-releasing position, wherein in the sleeve-unlocking position, the locking mechanism is allowed to move to the unlocked state and thereby cause the telescopic sleeve assembly to collapse into the second state, but the retraction-lock mechanism is kept in the retraction-lock state, in which the sharp is held in the insertion position, and wherein in the retraction-releasing position, the retraction-lock mechanism is allowed to move to the released state and thereby cause the sharp to move to the retracted state. In particular, the movable component may be a pivot arm which is pivotable from the sleeve-unlocking position to the retraction-releasing position.

Alternatively, instead of a retractable sharp, the sensor device may comprise a non-retractable insertion support on which the insertable portion is arranged, the insertion support having an insertion support tip configured for piercing the patient's skin.

Providing a non-retractable insertion support instead of a retractable sharp eliminates the need for a sharp retraction mechanism and thus considerably simplifies the patch-like medical device.

Ideally, the insertion support is made of a material with sufficient rigidity to puncture the patient's skin, e.g. preferably a metal or ceramics.

The insertion support may have a width or diameter that is larger than the insertable portion of the sensor device configured to measure the analyte concentration. Alternatively, the insertion support may have a width or diameter that is equal or narrower than the insertable portion of the sensor device configured to measure the analyte concentration.

Independently of whether the patch-like medical device comprises a retractable sharp or a non-retractable insertion support, the insertable portion of the sensor device configured to measure the analyte concentration may comprise a substrate made of a flexible material, for instance a liquid crystal polymer (LCP) or a polyimide like Kapton^{™}, which may be as thin as 50 µm.

In case the insertable portion comprises a set of electrodes, in particular a working electrode, a reference electrode and a counter electrode, or a working electrode and a combined reference/counter electrode, the electrodes may all be arranged on a single side of the substrate. Alternatively, the electrodes may be distributed on a front side and a rear side of the substrate.

The insertion support may comprise at least one measurement window to enable access to one or more of the electrodes. The measurement window may be a laser cut, drilled or punched opening in the insertion support and may have a size that is equal, larger or smaller than a size of the electrode. Alternatively, the measurement window may comprise more than one opening or may comprise a perforated area.

The insertable portion may be fixed to the insertion support by fixations means, such as a fixation layer comprising biocompatible glue, an adhesive or epoxy cement, or by double-sided tape.

Alternatively, the electrodes and the conductive traces may be disposed directly onto the insertion support by using printing technologies or physical/chemical vapor deposition technologies. If the insertion support is made from an electrically conductive material, the insertion support may be coated with a layer of insulating material such as parylene by chemical vapor deposition before the electrodes and the conductive paths are disposed onto the insertion support.

To enhance the functionality of the patch-like medical device and enable close-loop operation, i.e. to allow for both measuring and influencing the analyte concentration, preferably in response to the measurement, the patch-like medical device may further comprise a delivery needle fixedly positioned relative to the base, the delivery needle having a delivery needle tip which protrudes from the contact surface and which is configured for piercing the patient's skin, wherein the telescopic sleeve assembly extends beyond the delivery needle tip to prevent the delivery needle tip from piercing the patient's skin in the first state, and exposes the delivery needle tip to enable piercing the patient's skin in the second state.

The term "delivery needle" as used herein refers to any elongated sharp element configured to pierce the patient's skin and having a lumen for delivering a liquid substance, in particular insulin, through the patient's skin.

In some cases, the delivery needle used to infuse a fluid into the subcutaneous tissue needle may act as the insertion support for the insertable portion of the sensor device, i.e. the electrodes and the conductive traces may be disposed on an outer wall of the delivery needle.

The medical patch-like device may further comprise a reservoir configured to contain a liquid substance, preferably insulin. The delivery needle may be fluidically connected to the reservoir. The medical patch-like device may comprise a pump mechanism, in particular a piston pump with an electric motor drive, causing the liquid substance to be pumped from the reservoir through the delivery needle. The reservoir is preferably arranged in the first device part if the patch-like medical device comprises two parts as described above, the reservoir being preferably arranged on the side of the base facing away from the contact surface.

Having the delivery needle being fixedly positioned with respect to the contact surface of the base provides the advantage that the patient may comfortably move while wearing the patch-like medical device without causing a flow of the liquid substance to be inadvertently interrupted by an accidental movement of the delivery needle. Another advantage of a fixed needle is that it enables a fixed fluidic communication path from the reservoir to the delivery needle. No releasable fluidic connections between the reservoir and the delivery needle are needed if the needle is fixed, i.e. not movable with respect to the base, which reduces the risk of leakage. Furthermore, it allows delivery of the liquid substance at a pre-defined and constant distance with respect to the insertable portion of the sensor device.

In order to facilitate insertion in a case where the delivery needle and the insertable portion of the sensor device are separate from each other, the delivery needle preferably protrudes from the contact surface by a needle protrusion length which is shorter than a sharp length of the sharp in the insertion position, or than an insertion support length of the non-retractable insertion support. In particular, the sharp length or the insertion support length may be between 5 % and 50 % longer than the needle protrusion length.

Different aspects may have to be considered when a liquid substance is delivered in proximity to the insertable portion configured to measure the analyte concentration: a first aspect is that dilution effects may occur, i.e. the analyte concentration may be locally diluted by the subcutaneous delivery of the liquid substance. A second aspect is that interaction effects may occur, i.e. electrochemically active excipients present in the liquid substance may interact with the insertable portion of the sensor device, in particular via oxidation at the working electrode. Both effects can affect the measurement values obtained by the sensor device. However, both effects are quantifiable and may be compensated using a compensation routine, such as a compensation algorithm. The insertable portion of the sensor device and the delivery needle tip being in a fixed and defined geometrical relation to each other increases the reliability of said compensation routine. However, the liquid substance may propagate through the patient's tissue in an inhomogeneous manner due to local inhomogeneities in the patient's tissue. Thus, the less tissue there is between the insertable portion of the sensor device and the delivery needle tip, the lower the influence of such inhomogeneities, and thus the more predictable both the dilution effect and interaction effect become, which in turn enables a more reliable compensation routine.

In view of the considerations mentioned above, the insertable portion, in particular the working electrode, and the delivery needle tip are preferably spaced apart by a lateral distance of less than 5 mm, the lateral distance being defined in a plane parallel to the contact surface. Alternatively or additionally, the working electrode and the delivery needle tip are preferably spaced apart spaced apart by a longitudinal distance perpendicular to the contact surface of less than 5 mm.

Furthermore, the insertion support tip and the delivery needle tip preferably extend from the contact surface in parallel and form an angle of 90° with the contact surface to facilitate insertion and removal of the patch-like medical device after use.

In a second aspect, it is an object of the present invention to provide a patch-like medical device with a sensor device comprising an insertable portion configured to measure an analyte concentration subcutaneously which is particularly easy to insert, while allowing the insertable portion to be flexible to enhance the comfort for the patient.

A patch-like medical device is thus provided, comprising:
a base having a contact surface configured to be directed towards a patient's skin;
a sensor device comprising an insertable portion which is configured to measure an analyte concentration subcutaneously, wherein the insertable portion is fixedly positioned relative to the base and protrudes from the contact surface;
a sensor insertion mechanism comprising:
   a sharp having a sharp tip configured for piercing the patient's skin, the sharp being movable from an insertion position to a retracted position,
   wherein in the insertion position, the insertable portion of the sensor device is at least partially received within the sharp and the sharp tip extends beyond the insertable portion of the sensor device to enable insertion of the insertable portion into the patient's skin, and
   wherein in the retracted position, the insertable portion of the sensor device is exposed, and
   a retraction drive mechanism configured to drive the movement of the sharp from the insertion position to the retracted position.

The retraction drive mechanism may comprise a sharp retraction spring for driving the movement of the sharp, wherein in the insertion position, the sharp retraction spring is in a biased state.

Further statements made above with regards to the sharp in the context of the first aspect of the invention are applicable to a patch-like medical device according to the second aspect of the invention as well.

The sensor insertion mechanism may further comprise a retraction-lock mechanism having a retraction-lock state, in which the sharp is held in the insertion position, and a released state, in which the sharp is allowed to move to the retracted state. Furthermore, the sensor insertion mechanism may comprise a retraction trigger mechanism configured to switch the retraction-lock from the retraction-lock state to the released state.

The retraction-lock mechanism may in particular comprise a switch element to switch the retraction-lock from the retraction-lock state to the released state, wherein the switch element preferably comprises or consists of a shape-memory alloy.

Further statements made above with regards to the switch element in the context of the first aspect of the invention are applicable to a patch-like medical device according to the second aspect of the invention as well.

In a third aspect, it is an object of the present invention to provide a patch-like medical device with a sensor device having an insertable portion configured to measure an analyte concentration subcutaneously, which allows to additionally influence the analyte concentration, while being mechanically robust.

A patch-like medical device is thus provided, the patch-like medical device comprising:
a base having a contact surface configured to be directed towards a patient's skin;
a sensor device comprising:
   an insertable portion which is configured to measure an analyte concentration subcutaneously, and
   a non-retractable insertion support on which the insertable portion is arranged,
   wherein the insertable portion and the non-retractable insertion support are fixedly positioned relative to the base and protrude from the contact surface, and
   wherein the non-retractable insertion support has an insertion support tip configured for piercing the patient's skin, and
a delivery needle fixedly positioned relative to the base, the delivery needle having a delivery needle tip which protrudes from the contact surface and which is configured for piercing the patient's skin.

Having the delivery needle being fixedly positioned with respect to the contact surface of the base provides the advantage that the patient may comfortably move while wearing the patch-like medical device without causing a flow of the liquid substance to be inadvertently interrupted by an accidental movement of the delivery needle. Another advantage of a fixed needle is that it enables a fixed fluidic communication path from the reservoir to the delivery needle. No releasable fluidic connections between the reservoir and the delivery needle are needed if the needle is fixed, i.e. not movable with respect to the base, which reduces the risk of leakage. Furthermore, it allows delivery of the liquid substance at a pre-defined and constant distance with respect to the insertable portion of the sensor device.

Having the insertable portion of the sensor device being fixedly positioned relative to the base provides enhanced mechanical stability and facilitates electrical connectability, since there is no need for electrical wiring which has to be flexible or movable with respect to the base. Furthermore, it allows for the analyte concentration to be measured at a pre-defined distance from the contact surface, and thus from the surface of the patient's skin, and therefore does not depend on an insertion depth which may vary if the insertable portion of the sensor device is movable with respect to the base and the contact surface and may therefore inadvertently not always be inserted at the same depth. Providing a non-retractable insertion support eliminates the need for retraction mechanism and allows for a simpler medical device.

Combining both a fixed delivery needle and a fixed insertable portion of a sensor device on a non-retractable needle support thus leads to a mechanically particularly robust patch-like medical device.

The insertable portion the insertable portion, in particular the working electrode, and the delivery needle tip are preferably spaced apart by a lateral distance of less than 5 mm, the lateral distance being defined in a plane parallel to the contact surface. Alternatively or additionally, the working electrode and the delivery needle tip are preferably spaced apart spaced apart by a longitudinal distance perpendicular to the contact surface of less than 5 mm.

Furthermore, the insertion support tip and the delivery needle tip preferably extend from the contact surface in parallel and form an angle of 90° with the contact surface.

The statements made above regarding the non-retractable insertion support are applicable to a patch-like medical according to the third aspect of the invention as well.

The advantages described above regarding the positioning of the insertable portion of the sensor device and the delivery needle tip with respect to the base of the patch-like medical device, as well as regarding the positioning with respect to each other, are present in the patch-like medical device according to the third aspect of the invention as well.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1A: shows a perspective view of a patch-like medical device according to a first embodiment of the present invention with a telescopic sleeve assembly in a first state;
- Fig. 1B: shows a perspective view of the patch-like medical device shown in Fig. 1A with the telescopic sleeve assembly in a second state and with the sharp in an insertion position;
- Fig. 1C: shows a perspective view of the patch-like medical device shown in Fig. 1A with the telescopic sleeve assembly in the second state and with the sharp in a retracted position;
- Fig. 2A: shows a perspective view of the first device part of the patch-like medical device shown in Fig. 1A, i.e. wherein the second device part has been removed, and where the sharp is in the insertion position;
- Fig. 2B: shows a perspective view of the first device part of the patch-like medical device shown in Fig. 1A, i.e. with the second device part being removed, and with the sharp in the retracted position;
- Fig. 3A: shows a perspective sectional view of the first device part of the patch-like medical device shown in Fig. 2A where the reservoir and other elements have been removed to make a first example of the retraction trigger mechanism visible, with the telescopic needle assembly in the first state, with the sharp in the insertion position and with the pivot arm of the trigger mechanism in the first pivot position;
- Fig. 3B: shows the first device part patch-like medical device shown in Fig. 3A with the telescopic sleeve assembly in the second state, with the sharp in the insertion position and with the pivot arm of the trigger mechanism in the sleeve-unlocking position;
- Fig. 3C: shows the first device part patch-like medical device shown in Fig. 3A with the telescopic sleeve assembly in the second state, with the sharp moving in proximal direction and with the pivot arm in the retraction-releasing position;
- Fig. 3D: shows the first device part patch-like medical device shown in Fig. 3A with the telescopic sleeve assembly in the second state, with the sharp in the retracted position and with the pivot arm in a fourth position;
- Fig. 4A: shows a sectional view of the first device part patch-like medical device in the same state as shown in Fig. 3A, but with the sensor device removed for better visibility;
- Fig. 4B: shows a sectional view of the first device part patch-like medical device in the same state as shown in Fig. 3B, but without the sensor device for better visibility;
- Fig. 4C: shows a sectional view of the first device part patch-like medical device with the pivot arm in the retraction-releasing position and with the sharp retraction spring in a relaxed state, but without the sensor device for better visibility;
- Fig. 4D: shows a sectional view of the first device part patch-like medical device with the pivot arm in the fourth position and with the sharp retraction spring in a relaxed state, but without the sensor device for better visibility;
- Fig. 5A: shows a perspective sectional view of a second example of the retraction trigger mechanism with the retraction-lock mechanism in the retraction-lock state;
- Fig. 5B: shows a top view of the second example of the retraction trigger mechanism with the retraction-lock mechanism in the retraction-lock state;
- Fig. 5C: shows a perspective sectional view of the second example of the retraction trigger mechanism with the retraction-lock mechanism in the released state;
- Fig. 5D: shows a top view of the second example of the retraction trigger mechanism with the retraction-lock mechanism in the released state;
- Fig. 5E: shows a sectional view of the second example along the sectional plane marked in Fig. 5B;
- Fig. 5F: shows a sectional view of the second example along the sectional plane marked in Fig. 5D as the sharp retraction spring is about to expand;
- Fig. 5G: shows a sectional view of the second example along the sectional plane marked in Fig. 5D with the sharp in the retracted position;
- Fig. 6A: shows a perspective sectional view of a third example of the retraction trigger mechanism with the retraction-lock mechanism in the retraction-lock state;
- Fig. 6B: shows a top view of the third example of the retraction trigger mechanism with the retraction-lock mechanism in the retraction-lock state;
- Fig. 6C: shows a perspective sectional view of the third example of the retraction trigger mechanism with the retraction-lock mechanism in the released state;
- Fig. 6D: shows a top view of the third example of the retraction trigger mechanism with the retraction-lock mechanism in the released state;
- Fig. 6E: shows an enlarged perspective detail view of the switch strip in the first bent shape shown in Fig. 6A;
- Fig. 7A: shows a perspective sectional view of a fourth example of the retraction trigger mechanism with the retraction-lock mechanism in the retraction-lock state;
- Fig. 7B: shows a top view of the fourth example of the retraction trigger mechanism with the retraction-lock mechanism in the retraction-lock state;
- Fig. 7C: shows a perspective detail view of the fourth example of the retraction-lock mechanism in the retraction-lock state;
- Fig. 7D: shows a sectional view of the fourth example of the retraction trigger mechanism with the retraction-lock mechanism in the retraction-lock state along the sectional plane marked in Fig. 7B;
- Fig. 7E: shows a perspective sectional view of a fourth example of the retraction trigger mechanism with the retraction-lock mechanism in the released state;
- Fig. 7F: shows a top view of the fourth example of the retraction trigger mechanism with the retraction-lock mechanism in the released state;
- Fig. 7G: shows a perspective detail view of the fourth example of the retraction-lock mechanism in the released state;
- Fig. 7H: shows a sectional view of the fourth example of the retraction trigger mechanism with the retraction-lock mechanism in the released state along the sectional plane marked in Fig. 7F;
- Fig. 8A: shows perspective view of a patch-like medical device 1 according to a second embodiment of the present invention with the telescopic sleeve assembly in the first state;
- Fig. 8B: shows the second embodiment of the present invention with the telescopic shown in Fig. 8A with the telescopic sleeve assembly in the second state, exposing a delivery needle and the insertable portion of the sensor device on a non-retractable insertion support;
- Fig. 8C: shows an enlarged view of the insertable portion of the sensor device on the non-retractable insertion support and a delivery needle shown in Fig. 8B;
- Fig. 9A: shows a front view of a first example of a non-retractable insertion support together with the insertable portion of the sensor device;
- Fig. 9B: shows a rear view of the first example of a non-retractable insertion support together with the insertable portion of the sensor device;
- Fig. 9C: shows a sectional view of the first example of a non-retractable insertion support together with the insertable portion of the sensor device along the sectional plane marked in Fig. 9A;
- Fig. 10A: shows a front view of a second example of a non-retractable insertion support together with the insertable portion of the sensor device;
- Fig. 10B: shows a rear view of the second example of a non-retractable insertion support together with the insertable portion of the sensor device;
- Fig. 10C: shows a sectional view of the second example of a non-retractable insertion support together with the insertable portion of the sensor device along the sectional plane marked in Fig. 10B;
- Fig. 11A: shows a front view of a third example of a non-retractable insertion support together with the insertable portion of the sensor device;
- Fig. 11B: shows a rear view of the third example of a non-retractable insertion support together with the insertable portion of the sensor device;
- Fig. 11C: shows a sectional view of the third example of a non-retractable insertion support together with the insertable portion of the sensor device along the sectional plane marked in Fig. 11A;
- Fig. 12A: shows a front view of a fourth example of a non-retractable insertion support together with the insertable portion of the sensor device;
- Fig. 12B: shows a rear view of the fourth example of a non-retractable insertion support together with the insertable portion of the sensor device;
- Fig. 12C: shows a sectional view of the fourth example of a non-retractable insertion support together with the insertable portion of the sensor device along the sectional plane marked in Fig. 12A;
- Fig. 13A: shows a front view of a fifth example of a non-retractable insertion support together with the insertable portion of the sensor device;
- Fig. 13B: shows a rear view of the fifth example of a non-retractable insertion support together with the insertable portion of the sensor device;
- Fig. 13C: shows a sectional view of the fifth example of a non-retractable insertion support together with the insertable portion of the sensor device along the sectional plane marked in Fig. 13A;
- Fig. 14A: shows a front view of a sixth example of a non-retractable insertion support together with the insertable portion of the sensor device;
- Fig. 14B: shows a rear view of the sixth example of a non-retractable insertion support together with the insertable portion of the sensor device;
- Fig. 14C: shows a sectional view of the sixth example of a non-retractable insertion support together with the insertable portion of the sensor device along the sectional plane marked in Fig. 14B;
- Fig. 15A: shows a front view of a seventh example of a non-retractable insertion support together with the insertable portion of the sensor device;
- Fig. 15B: shows a rear view of the seventh example of a non-retractable insertion support together with the insertable portion of the sensor device;
- Fig. 15C: shows a sectional view of the seventh example of a non-retractable insertion support together with the insertable portion of the sensor device along the sectional plane marked in Fig. 15A;
- Fig. 16A: shows a front view of an eighth example of a non-retractable insertion support together with the insertable portion of the sensor device;
- Fig. 16B: shows a rear view of the eighth example of a non-retractable insertion support together with the insertable portion of the sensor device;
- Fig. 16C: shows a sectional view of the eighth example of a non-retractable insertion support together with the insertable portion of the sensor device along the sectional plane marked in Fig. 16B;
- Fig. 17A: shows a front view of a ninth example of a non-retractable insertion support together with the insertable portion of the sensor device;
- Fig. 17B: shows a rear view of the ninth example of a non-retractable insertion support together with the insertable portion of the sensor device;
- Fig. 17C: shows a sectional view of the ninth example of a non-retractable insertion support together with the insertable portion of the sensor device along the sectional plane marked in Fig. 17A;
- Fig. 18A: shows a front view of a tenth example of a non-retractable insertion support together with the insertable portion of the sensor device;
- Fig. 18B: shows a rear view of the tenth example of a non-retractable insertion support together with the insertable portion of the sensor device, and
- Fig. 18C: shows a sectional view of the first example of a non-retractable insertion support together with the insertable portion of the sensor device along the sectional plane marked in Fig. 18B.

### DESCRIPTION OF PREFERRED EMBODIMENTS

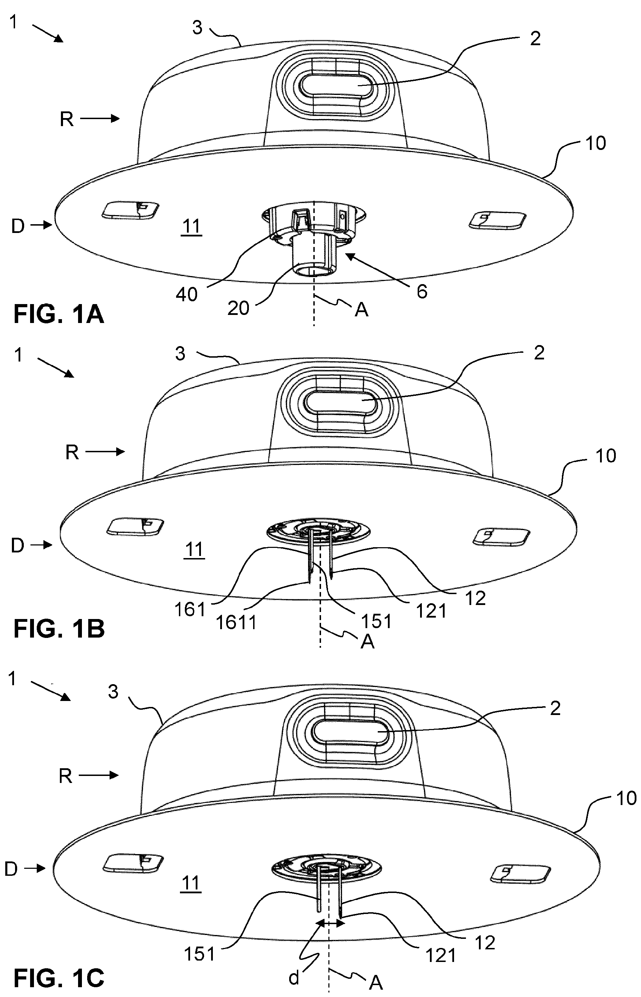

Figs. 1A to 4D illustrate a patch-like medical device according to a first embodiment of the present invention. The patch-like medical device according to this first embodiment is configured to both deliver a fluid through a patient's skin, as well as to subcutaneously measure a concentration of an analyte.

Figs. 1A to 1C show a perspective view of the patch-like medical device 1. The patch-like medical devices 1 comprises a first device part D and a second device part R, wherein the first device part D and the second device part R are configured to be releasable coupled to each other. The first device part comprises a base 10, which acts as a housing lower part, and the second device part comprises the housing upper part 3, which is configured to be releasably coupled to the base 10. Preferably, the first device part D is disposable and the second device part R is reusable with another first device part D of the same kind. The base 10 has a contact surface 11 configured to be directed towards a patient's skin. Said contact surface 11 may comprise an adhesive layer or may be configured to be attached to an adhesive plaster to enable the patch-like medical device to be worn by the patient over an extended period of time.

The patch-like medical device 1 comprises a sensor device 15, the sensor device 15 comprising an insertable portion 151 which is configured to subcutaneously measure an analyte concentration, in particular a glucose concentration. In particular, the insertable portion 151 may comprise a set of electrodes which are configured to measure the analyte concentration in an electrochemical manner. The insertable portion 151 is fixedly positioned relative to the base 10 and protrudes from the contact surface 11.

Furthermore, the patch-like medical device comprises a delivery needle 12 fixedly positioned relative to the base 10. The delivery needle 12 has a needle tip 121 which protrudes from the contact surface 11 and which is configured for piercing the patient's skin. In the embodiments shown here, the delivery needle 12 is a cannula with a lumen to deliver a liquid substance, e.g. a formulation containing insulin, through the patient's skin.

However, in an alternative embodiment, the patch-like medical device may be configured to subcutaneously measure a concentration of an analyte without providing means for fluid delivery within the same device, and therefore would not comprise the delivery needle 12.

To protect the patient, to protect both the insertable portion 151 of the sensor device 15 and the delivery needle tip 121, and to facilitate their insertion into the patient's skin, the patch-like medical device comprises a telescopic sleeve assembly which is collapsible along a central axis A from a first state into a second state. In the first state, the telescopic sleeve assembly extends beyond the insertable portion 151 of the sensor device 15 and beyond the delivery needle tip 121.

Fig. 1A shows the telescopic sleeve assembly in the first state. In Figs. 1B and 1C, the telescopic sleeve assembly is the second state. In this second state, the telescopic sleeve assembly is arranged inside the patch-like medical device 1 and does not protrude from the contact surface 11.

In the embodiment shown in Figs. 1A to 4D, the patch-like medical device comprises a sensor insertion mechanism 16 which comprises a sharp 161 having a sharp tip 1611 to enable insertion of the insertable portion 151 into the patient's skin. The sharp 161 is movable from an insertion position, shown in Fig. 1B, to a retracted position, shown in Fig. 1C. In the insertion position, the insertable portion 151 of the sensor device 15 is at least partially received within the sharp 161 and the sharp tip 1611 extends beyond the insertable portion 151 of the sensor device to enable insertion of the insertable portion into the patient's skin. In the retracted position, the insertable portion 151 of the sensor device 15 is exposed. Buttons 2 are arranged on the housing upper part 3 to operate the patch-like medical device 1, in particular to initiate the collapse of the telescopic needle assembly 6 and/or the retraction of the sharp 161.

In order to facilitate insertion, the delivery needle preferably protrudes from the contact surface by a needle protrusion length which is shorter than a sharp length of the sharp. In a specific embodiment, the needle protrusion length is 6.1 mm, the insertable portion length of the insertable portion of the sensor device is between 5 mm and 5.5 mm, and the sharp has a sharp length of 6.8 mm, wherein all lengths mentioned in this sentence are measured from the contact surface 11.

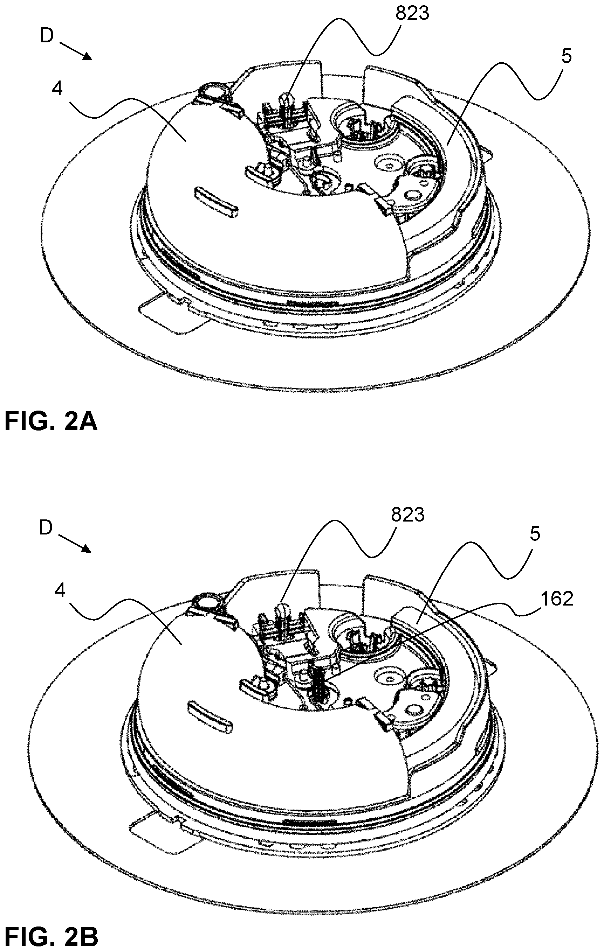

Figs. 2A and 2B show a perspective view of the first device part D of patch-like device 1 of Figs. 1A-1C, i.e. where the second device part R has been removed. The first device part D comprises a reservoir assembly with a reservoir 4 configured to contain a liquid substance to be administered to the patient, as well as a piston 5 to move the liquid substance within the reservoir 4, in particular to move the liquid substance towards an exit end of the reservoir 4, which is fluidically connected to the delivery needle 12. To enable a compact design, the reservoir 4 has a toroidal shape and the piston 5 has a piston arm that is curved accordingly in order to fit into the reservoir 4. Preferably, the piston 5 comprises a piston head having a cross-section matching the cross-section of the reservoir 4 and which comprise a seal element surrounding the piston head, the seal element gliding along an inner wall of the reservoir 4 when the piston head is moved backward and forward, thereby sealing the reservoir 4 at one end.

The sensor insertion mechanism comprises a retraction drive mechanism configured to drive the movement of the sharp 161 from the insertion position to the retracted position. In this first embodiment, the retraction drive mechanism comprises a sharp retraction spring 162 for driving the movement of the sharp 161, wherein in the insertion position, and sharp retraction spring 162 is in a biased state. The sharp retraction spring 162 extends in parallel to the central axis A. In Fig. 2A, which corresponds to a situation in which the sharp 161 is in the insertion position, sharp retraction spring 162 is not visible, as it is concealed by surrounding elements. In Fig. 2B however, which corresponds to a situation in which the sharp 161 is in the retracted position, the sharp retraction spring 162 is visible, as it is in an extended state in which it extends beyond the surrounding elements.

The sensor insertion mechanism further comprises a retraction-lock mechanism having a retraction-lock state, in which the sharp 161 is held in the insertion position, and a released state, in which the sharp 161 is allowed to move to the retracted state. Furthermore, the sensor insertion mechanism comprises a retraction trigger mechanism configured to switch the retraction-lock from the retraction-lock state to the released state.

The retraction trigger mechanism may be implemented in different ways, as will be discussed in the following.

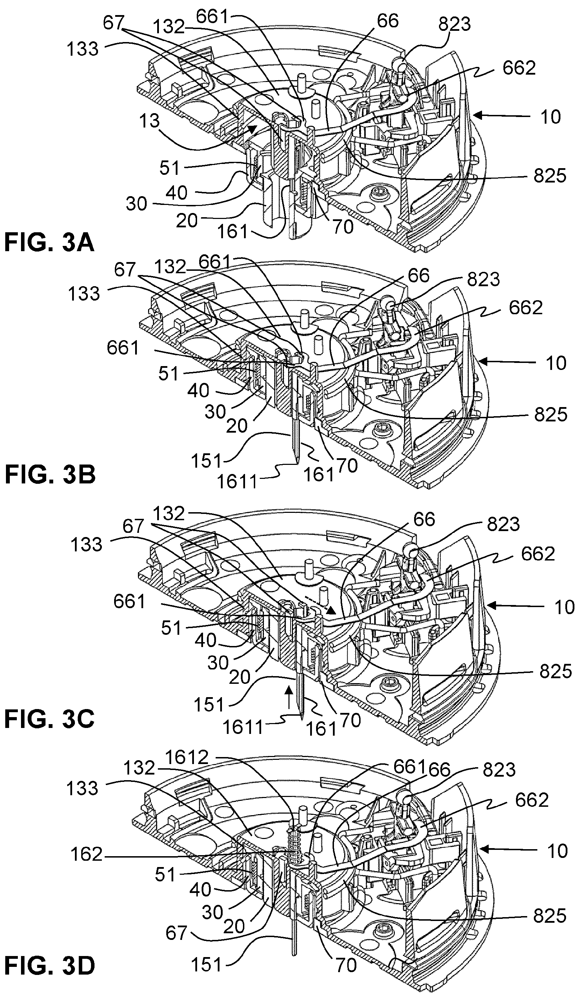

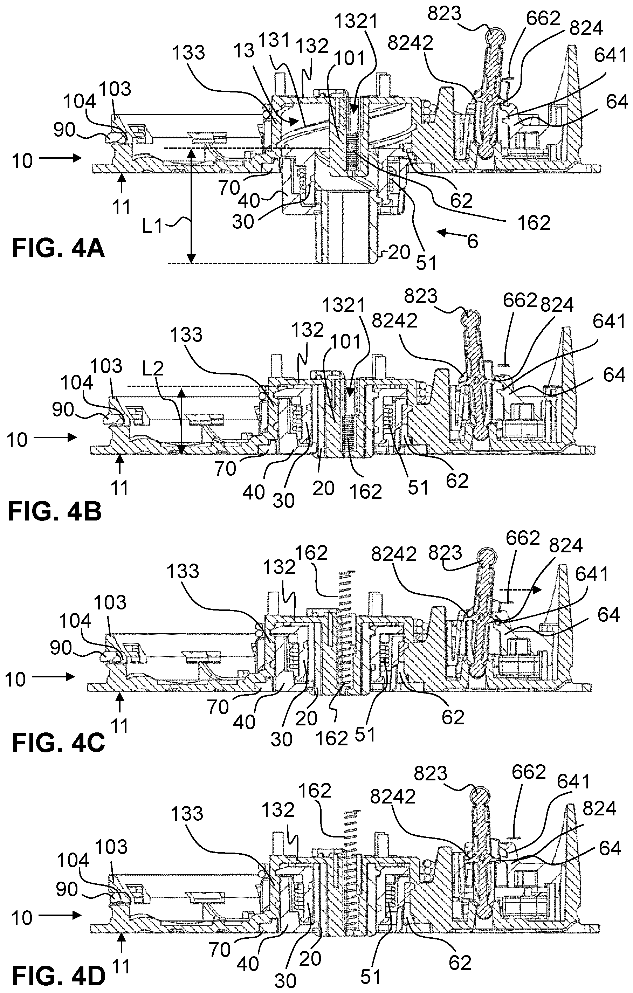

A first example of the retraction trigger mechanism is illustrated in Figs. 3A to 4D. Figs. 3A to 3D show a perspective sectional view of the first device part D of the patch-like medical device 1, where the reservoir 4 and other elements have been removed to make the retraction trigger mechanism visible. Figs. 4A to 4D show corresponding sectional views of the first device part D, wherein the sharp and the insertable portion of the sensor device have been removed for further illustrative simplicity.

In this first example, the retraction trigger mechanism for retracting the sharp 161 is configured to cooperate with a different trigger mechanism, namely a trigger mechanism which is used to trigger the collapse of the telescopic sleeve assembly 6 from its first state to its second state. A similar trigger mechanism to trigger the collapse of the telescopic sleeve assembly 6 from its first state to its second state is known from WO2023198343A1, which is incorporated herein by reference in its entirety. To provide mechanical control over the collapse of the telescopic sleeve assembly 6, the patch-like medical device 1 comprises a locking mechanism having a locked state and an unlocked state. In the locked state, the locking mechanism prevents the telescopic sleeve assembly 6 from collapsing into the second state. In the unlocked state, the locking mechanism allows the telescopic sleeve assembly 6 to collapse into the second state. The trigger mechanism is configured to switch the locking mechanism from the locked state to the unlocked state. The trigger mechanism for switching the locking mechanism from the locked state to the unlocked state and the retraction trigger mechanism for switching the retraction-lock mechanism from the retraction-lock state to the released state share a movable component.

In the following, the embodiment of the telescopic sleeve assembly 6, the locking mechanism and the trigger mechanism shown in Figs. 3A to 4D is described in more detail.

The telescopic sleeve assembly 6 comprises a protection sleeve 20, a rotating sleeve 30, a rotation lock sleeve 40, and a fixation element in form of a fixation ring 70. The protection sleeve 20 is movable along the central axis A from a first position in the first state to a second position in the second state. The rotating sleeve 30 is movable along the central axis A and is in a thread-like engagement with the protection sleeve 20 such that a helical movement of the rotating sleeve 30 with respect to the base 10 causes the protection sleeve 20 to move along the central axis A from the first position to the second position. The rotating sleeve 30 is in a thread-like engagement with a side wall 133 that delimits the recess 13. The thread-like engagement of the protection sleeve 20 with the rotating sleeve 30 exhibits a handedness that is opposite to the handedness of the thread-like engagement of the rotating sleeve 30 with the side wall 133 that delimits the recess 13. The side wall 133 comprises an internal thread 131 with which the rotating sleeve 30 is in engagement. The rotation lock sleeve 40 is movable along the central axis A while being rotationally fixed with respect to the base 10. The rotation lock sleeve 40 is in engagement with the protection sleeve 20 such that the protection sleeve 20 is prevented from rotating with respect to the base 10 when the protection sleeve 20 moves from the first position to the second position. The fixation ring 70 is configured to prevent the rotation lock sleeve 40 from rotating with respect to the base 10.

The base 10 defines a recess 13 arranged on a side of the base 10 being opposite to the contact surface 11. The recess is delimited in axial direction by a top wall 132. In radial direction, the recess 13 is delimited by a cylindrical side wall 133.

The telescopic sleeve assembly 6 further comprises a spring 51. Preferably, the spring 51 is a helical torsion spring capable of exerting a twisting force. The spring 51 has a first end and a second end, wherein the first end is connected to the rotation lock sleeve, and the second end is connected to the rotating sleeve 30. The spring 51 is wrapped around the rotating sleeve 30 between an outer wall of the rotating sleeve and an inner wall of the rotation lock sleeve 40.

In Fig. 3A and Fig. 4A, the telescopic sleeve assembly 6 is in the first state, in which it has a first assembly length L1 in direction of the central axis A from a proximal end of the rotating sleeve 30 to a distal end of the protection sleeve 20. The spring 51 is pre-loaded and in particular exerts a twisting force between the rotating sleeve 30 and the rotation lock sleeve 40. Since the rotation lock sleeve 40 is prevented from rotating by the fixation ring 70, the rotating sleeve 30 is urged to carry out a helical movement in proximal direction. To prevent the rotating sleeve 30 from moving prior to use, the rotating sleeve 30 is held back by a latch 62, which is part of the locking mechanism. The locking mechanism further comprises a lever element (not visible) connected to the latch 62 and a clamp element 64 configured to clamp the lever element. The latch 62 is pivotably connected to the base 10. In the locked state, the latch 62 is engaged with the rotating sleeve 30 to prevent the rotating sleeve 30 from moving. In the unlocked state, the latch 62 is disengaged from the rotating sleeve 30 to allow the rotating sleeve 30 to carry out the helical movement. The clamp element 64 can be mechanically actuated to move from a first clamp position to a second clamp position. By moving from the first clamp position to the second clamp position, the clamp element 64 releases the lever element, allowing it to move from a first lever element position to a second lever element position as the latch pivots outwards and disengages from the rotating sleeve 30, the rotating sleeve 30 being now free to perform the helical movement causing the protection sleeve 20 to move from the first position to the second position.

The trigger mechanism comprises an actuation mechanism with a pivot arm 823. The pivot arm 823 is pivotable from a first pivot position (Fig. 3A and Fig. 4A) to a second pivot position (Fig. 3B and Fig. 4B), the second pivot position corresponding to a sleeve-unlocking position, in which the locking mechanism is allowed to move to the unlocked state and thereby cause the telescopic sleeve assembly 6 to collapse into the second state, but the retraction-lock mechanism is kept in the retraction-lock state, in which the sharp 161 is held in the insertion position. In the second state, the telescopic sleeve assembly 6 has a second assembly length L2 in direction of the central axis A from a proximal end of the rotating sleeve 30 to a distal end of the protection sleeve 20, which is shorter than the first assembly length L1. The telescopic sleeve assembly 6 is thus space-saving when it is fully retracted inside the recess 13 of the base 10.

The pivot arm 823 has a nose-like protrusion 824, which in the first pivot position pushes the clamp element 64 in distal direction (and thereby prevents it from moving) against a force exerted on the clamp element 64 by a resilient wire arrangement 825. The resilient wire arrangement 825 is partially wrapped around the sidewall 133 delimiting the recess 13 and urges the clamp element 64 to move in proximal direction from the first clamp position (where the clamp element 64 clamps the lever element) to the second clamp position (where the lever element is released).

When the pivot arm 823 is being moved to the second pivot position (Fig. 3B and Fig. 4B), the clamp element 64 moves in proximal direction, being linearly guided by a guiding structure that is arranged on the base 10. As the clamp element 64 moves in proximal direction, it releases the lever element, which then pivots from the first lever element position to the second lever element position as the latch 62 is pushed radially outward by the rotating sleeve 30 that is urged to rotate by the pre-loaded spring 51.

While the spring 51 at least partially relaxes, the rotating sleeve 30 performs the helical movement, i.e. in this case screws itself into the recess 13 while dragging along the rotation lock sleeve 40, which is hooked to a distal rim 39 of the rotating sleeve via three hooks. The dragged rotation lock sleeve 40 performs a linear movement in proximal direction, as it is prevented from rotating by the fixation ring 70, which in turn is attached to the base 10. The protection sleeve 20, which is prevented from rotating by the rotation lock sleeve 40, linearly move towards the base 10 while effectively getting screwed into the rotating sleeve 30, since the protection sleeve 20 is in a thread-like engagement with the rotating sleeve 30.

Ultimately, the protection sleeve 20 reaches its second position, i.e. the telescopic sleeve assembly 6 reaches its second state, shown in Fig. 3B and Fig. 4B, in which the telescopic sleeve assembly 6 has a second assembly length L2 in direction of the central axis A that is shorter than the first assembly length L1. In this second state, the sharp tip 161 is exposed and all the sleeves are fully retracted inside the recess 13 of the base 10. In Fig. 3B and Fig. 4B, the clamp element 64 is in the second clamp position. In this second clamp position, the clamp element 64 is being pushed in distal direction by a second nose-like protrusion 8242 of the pivot arm 823 arranged radially opposite to the first nose-like protrusion 824 against the force exerted on the clamp element 64 by the resilient wire arrangement 825.

The trigger mechanism for the telescopic sleeve assembly 6 and the retraction trigger mechanism for retracting the sharp 161 share the pivot arm 823.

The retraction-lock mechanism comprises a retraction-lock arm 66, which is configured to hold the sharp 161 in the insertion position. In particular, the retraction-lock arm 66 has a first end 661 which is U-shaped, as visible in Figs. 3A to 3D, and which is arranged on a proximal side of the top wall 132 of the recess 13. An axial protrusion 101 extends distally from the top wall 132 into the recess 13. The sharp retraction spring 162 is arranged in an axial guiding bore 1321 formed within the axial protrusion 101. The sharp 161 has a proximal sharp end 1612 opposite the distal sharp tip 1611, the proximal sharp end 1612 being in engagement with a proximal end of the sharp retraction spring 162. The retraction-lock mechanism comprises a pair of push-down pins 67 which are arranged within the guiding bore 1321 and extend in proximal direction. The push-down pins are arranged diametrically opposite each other in the guiding bore 1321. The push-down pins 67 have distal ends which hold the sharp retraction spring 162 it its biased state, i.e. in particular which hold the sharp retraction spring 162 down in a compressed state. The push-down pins 67 have proximal ends which are urged to emerge from the guiding bore on a proximal side of the top wall 132 by the sharp retraction spring 162.

In Figs. 3A and 3B, and in Figs. 4A and 4B, the retraction-lock mechanism is in the retraction-lock state. In the retraction-lock state, the U-shaped first end 661 of the retraction-lock arm exerts a force in distal direction on the proximal ends of the push-down pins 67, thereby preventing the push-down pins 67 to move in proximal direction, in particular to move beyond the proximal side of the top wall 132, and thus preventing the sharp retraction spring 162 from extending in proximal direction and retracting the sharp 161.

In Fig. 3C and Fig. 4C, the pivot arm 823 is in a third pivot position, which corresponds to a retraction-releasing position, in which the retraction-lock mechanism is allowed to move to the released state and thereby cause the sharp 161 to move to the retracted state. The retraction-lock arm 66 has a second end 662 which interacts with the pivot arm 823. As the pivot arm 823 is pivoted to the retraction-releasing position, it laterally displaces the second end 662 of the retraction-lock arm 66 in a plane parallel to the contact surface 11, as indicated by the dotted arrow in Fig. 4C. This causes the first end 661 of the retraction-lock arm 66 to release the distal ends of the push-down pins 67. Thus, the push-down pins 67 no longer hold the sharp retraction spring 162 down and the sharp retraction spring 162 can relax in proximal direction and thereby pull the sharp 161 out of the patient's skin, to expose the insertable portion 151 of the sensor device 15, as shown in Fig. 3D.

In the third position, the nose-like protrusion 824 of the pivot arm 832 engages with a clamp notch 641 of the clamp element 64, thus preventing the clamp element 64 from moving further in proximal direction.

In Fig. 4D, the pivot arm 832 is in a fourth position, in which the nose-like protrusion 824 is disengaged from the clamp element 64 and in which the clamp element 64 is displaced in proximal direction compared to the third position. By moving from the third to the fourth position, an open retaining ring 90, which is used to couple the base 10 to the housing upper part 3, is reduced in diameter, such that the open retaining ring 90 no longer protrudes from a circumferential base wall 103 of the base 10, but instead is retraced inside a base wall groove 104, which is formed along at least a portion of the base wall 103 in an outer surface of the base wall facing radially outwards. Retraction of the open retaining ring 90 into the base wall groove 104 allows the first device part D and the second device part R to be decoupled, so that they can be separated.

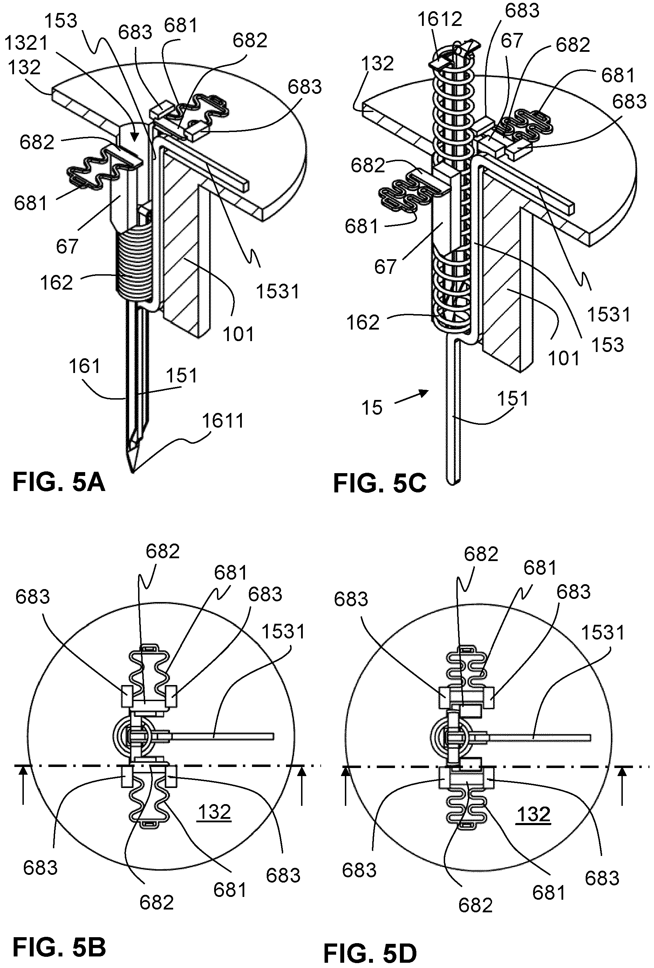

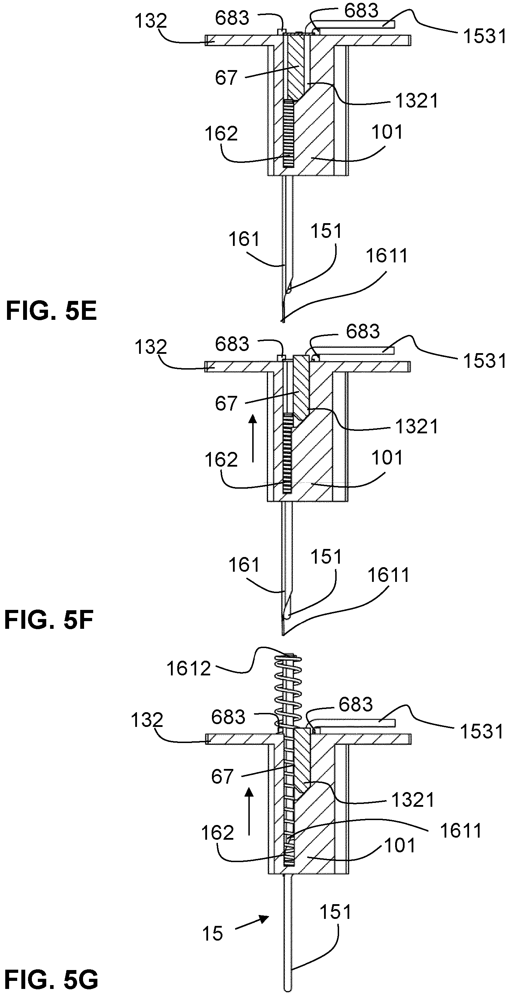

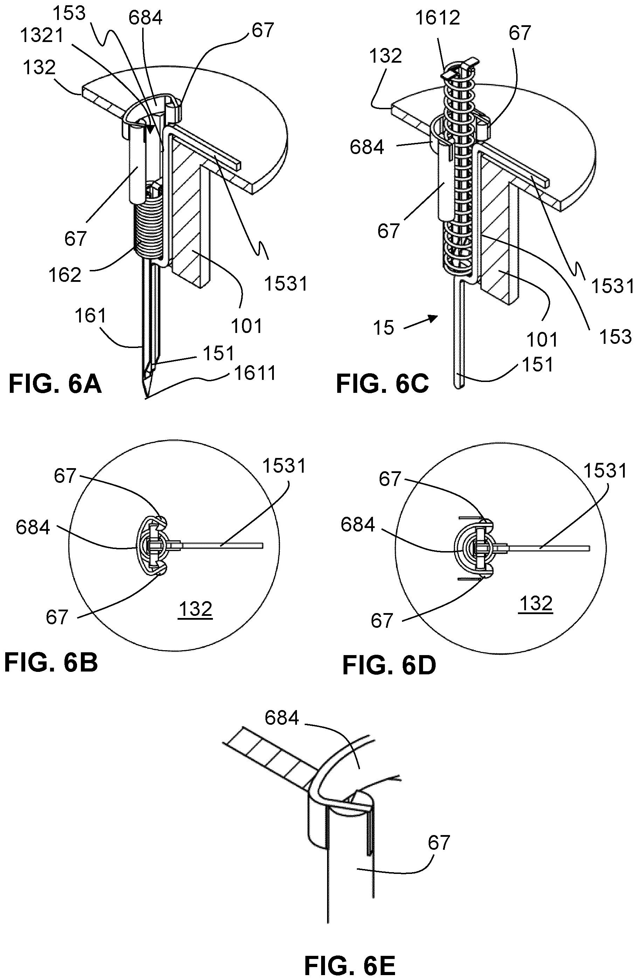

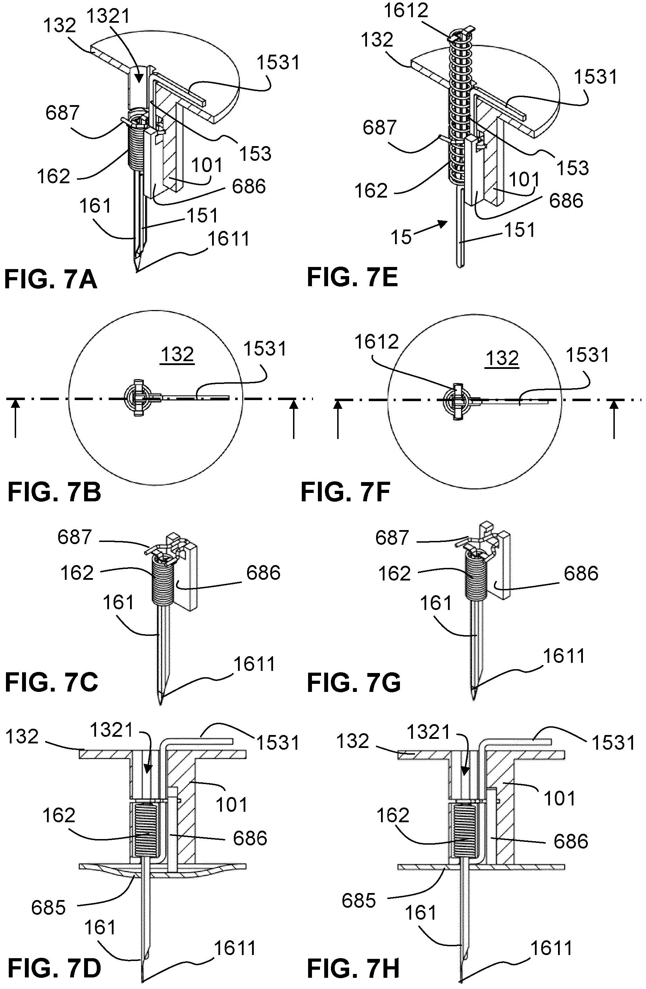

A second example of the retraction trigger mechanism is illustrated in Figs. 5A to 5G, a third example of the retraction trigger mechanism is illustrated in Figs. 6A to 6E, and a fourth example of the retraction trigger mechanism is illustrated in Figs. 7A to 7H. Each of these examples may be integrated in a patch-like medical device 1 like the one shown in Figs. 1A to 1C.

In this second, third and fourth example, the retraction trigger mechanism is independent from the trigger mechanism causing the collapse of the telescopic sleeve assembly 6. Thus, for the sake of simplicity, only the axial protrusion 101 of the base 10, which extends distally from the top wall 132 into the recess 13 and which contains the guiding bore 1321 are shown for these examples.

In the second, third and fourth example of the trigger mechanism, the trigger mechanism comprises a switch element 68 to switch the retraction-lock mechanism from the retraction-lock state to the released state. In the embodiments shown in Fig. 5A-7H, the sharp has a U-shaped profile, in which the insertable portion 151 of the sensor device is received. The sensor device 151 comprises a non-insertable portion 153, which extends through the guiding bore 1321 and comprises a contact portion 1531 (only schematically drawn), which is connectable to electronic circuitry for operation of the sensor device 15.

In the second example (Figs. 5A to 5G) and the third example (Figs. 6A to 6E), the switch element is at least partially made of a shape-memory alloy, a metal alloy of nickel and titanium, e.g. nitinol, or a copper (Cu)-based alloy.

In the second example, the switch element 68 comprises two wire loops 681 made of the shape-memory alloy, each wire loop 681 exhibiting sinusoidal bends along their circumferences and being attached to a switch plate 682. The bends are formed in a common contraction plane and allow the wire loops 681 to contract and expand in said contraction plane in a spring-like manner.

In Fig. 5A (perspective sectional view), Fig. 5B (top view) and Fig. 5E (sectional view), the switch element 68 is in an expanded state, which corresponds to the retraction-lock state of the retraction-lock mechanism, in which the sharp 161 is held in the insertion position.

Similar to the first example described above, two push-down pins 67 are arranged within the guiding bore 1321. The two wire loops 681 and the switch plates 682 to which they are attached are arranged diametrically opposite each other around the guiding bore 1321 on the proximal side of the top wall 132 of the recess 13. Two switch plate guiding structures 683 configured to each receive one of the switch plates 682 are fixedly arranged on the proximal side of the top wall 132. When the switch element 68 is the expanded state, one switch plate 682 is arranged on each of the distal end of the push-down pins 67 to prevent the push-down pins 67 from releasing the sharp retraction spring 162 arranged in the guiding bore 1321. The switch plates 682 are prevented from being displaced in proximal direction by being held within their respective switch plate guiding structure 683.

In order to active the switch element 68, the wire loops 681 are heated, e.g. by causing a DC-current to flow through the wire loops 681, which causes the wire loops 681 to contract. This contraction in turn causes the switch plates 682 to slide out of their switch plate guiding structures 683 parallel to the top wall 132, thereby releasing the push-down pins 67. As a result, the push-down pins 67 disengage from the sharp retraction spring 162, as shown in Fig. 5F, and the sharp retraction spring 162 extends in proximal direction, thereby pulling the sharp 161 out of the patient's skin.

In Fig. 5C (perspective sectional view) and Fig. 5D (top view), the switch element 68 is in a contracted state, which corresponds to the released state of the retraction-lock mechanism, in which the sharp 161 is retracted and the insertable portion 151 of the sensor device 15 is exposed.

In the third example, the switch element 68 comprises a switch strip 684 made of a shape-memory alloy.

In Fig. 6A (perspective sectional view) and Fig. 6B (top view), the switch element 68 is in a first state, which corresponds to the retraction-lock state of the retraction-lock mechanism, in which the sharp 161 is held in the insertion position. Similar to the first and second example described above, two push-down pins 67 are arranged within the guiding bore 1321. In this third example however, the distal ends of the push-down pins 67 are connected by the switch strip 684. In particular, each end of the switch strip 684 may be received within a slot formed at the distal end of each push-down pin 67, as visible in the enlarged view shown in Fig. 6F. In this first state, the switch strip 684 exhibits a first bent shape, which is designed such that the push-down pins 67, to which the switch strip 684 is attached, are in engagement with the sharp retraction spring 162.

In Fig. 6C (perspective sectional view) and Fig. 6D (top view), the switch element 68 is in a second state, which corresponds to the released state of the retraction-lock mechanism, in which the sharp 161 is retracted and the insertable portion 151 of the sensor device 15 is exposed.

In order to active the switch element 68, the switch strip 684 is heated, e.g. by causing a DC-current to flow through the switch strip 684, which causes the switch strip 684 to switch to a second bent shape, in which the ends of the switch strip 684 are further apart from each other than in the first bent shape. As the ends of the switch strip 684 move away from each other, the push-down pins 67 are pushed radially outwards. As a result, the push-down pins 67 disengage from the sharp retraction spring 162, allowing the sharp retraction spring 162 to extend in proximal direction and to thereby pull the sharp 161 out of the patient's skin.

In the fourth example, the switch element 68 comprises a push button 685, a sliding piece 686 and a resilient switch wire 687. The sliding piece 686 is arranged in a recess formed in a wall of the guiding bore 1321 so as to be slidable in axial direction. The resilient switch wire 687 is deformable from a squeezed state to a relaxed state.

In Fig. 7A (perspective sectional view), Fig. 7B (top view), Fig. 7C (perspective sectional view without the protrusion of the base) and Fig. 7D (sectional view), the switch element 68 is in a first state, which corresponds to the retraction-lock state of the retraction-lock mechanism, in which the sharp 161 is held in the insertion position. In this first state, the resilient switch wire 687 is held in its squeezed state by being in engagement with the sliding piece 686, and thereby exerts a force on the sharp retraction spring 162 in proximal direction, to prevent the sharp retraction spring 162 from extending in proximal direction.

In this first state, the push button 685 protrudes from the contact surface 11 of the base 10, such that when the patch-like medical device is applied to the patient's skin, the patient's skin exerts a force on the push button 685 in proximal direction.

As the push button 685 is pushed in proximal direction, the sliding piece 686, which is connected at its distal end to the push button 685, moves in proximal direction, and thereby releases the resilient switch wire 687 from its squeezed state, which causes the resilient switch wire 687 to release the sharp retraction spring 162, allowing the sharp retraction spring 162 to extend in proximal direction and to thereby pull the sharp 161 out of the patient's skin.

In Fig. 7E (perspective sectional view) and Fig. 7F (top view), the switch element 68 is in a second state, which corresponds to the released state of the retraction-lock mechanism, in which the sharp 161 is retracted and the insertable portion 151 of the sensor device 15 is exposed. Fig. 7G (perspective sectional view without the protrusion of the base) and Fig. 7H (sectional view), show an intermediate instant between the first and second state, where the push button has been depressed and is now flush with the contact surface 11 of the base and the resilient switch wire 687 is in the process of releasing the sharp retraction spring 162 by relaxing into its relaxed state.

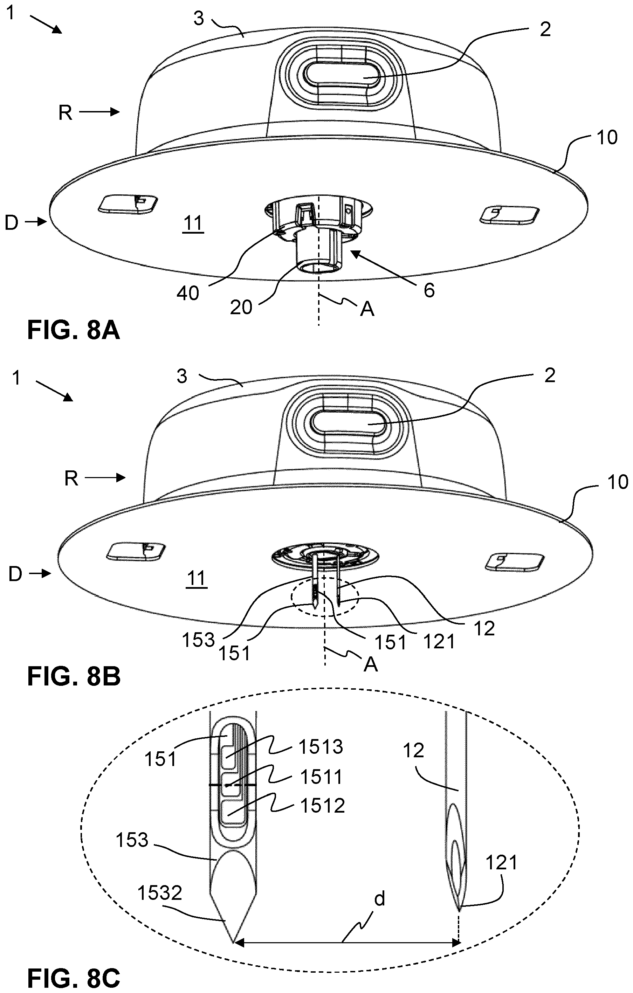

Figs. 8A to 8C show perspective views of a patch-like medical device 1 according to a second embodiment of the present invention. The patch-like medical device according to this first embodiment is configured to both deliver a fluid through a patient's skin, as well as to subcutaneously measure a concentration of an analyte.

The patch-like medical device 1 comprises a first device part D and a second device part R, wherein the first device part D and the second device part R are configured to be releasable coupled to each other. The first device part comprises a base 10, which acts as a housing lower part, and the second device part comprises the housing upper part 3, which is configured to be releasably coupled to the base 10. Preferably, the first device part D is disposable and the second device part R is reusable with another first device part D of the same kind. The base 10 has a contact surface 11 configured to be directed towards a patient's skin. Said contact surface 11 may comprise an adhesive layer or may be configured to be attached to an adhesive plaster to enable the patch-like medical device to be worn by the patient over an extended period of time.

The patch-like medical device shown in Figs. 8A to 8C comprises a delivery needle 12 fixedly positioned relative to the base 10. The delivery needle 12 has a needle tip 121 which protrudes from the contact surface 11 and which is configured for piercing the patient's skin. In the embodiments shown here, the delivery needle 12 is a cannula with a lumen to deliver a liquid substance, e.g. a formulation containing insulin, through the patient's skin.

However, in an alternative embodiment, the patch-like medical device may be configured to subcutaneously measure a concentration of an analyte without providing means for fluid delivery within the same device, and therefore would not comprise the delivery needle 12.

The patch-like medical device 1 according to this second embodiment comprises a sensor device 15, the sensor device 15 comprising an insertable portion 151 which is configured to subcutaneously measure an analyte concentration, in particular a glucose concentration. In particular, the insertable portion 151 comprises a set of electrodes 1511,1512,1513 which are configured to measure the analyte concentration in an electrochemical manner. The insertable portion 151 is fixedly positioned relative to the base 10 and protrudes from the contact surface 11.

In contrast to the first embodiment shown in Figs. 1A-1C, the patch-like medical device 1 according to this second embodiment does not comprise a retractable sharp. Instead, the sensor device comprises a non-retractable insertion support 152 on which the insertable portion 151 is arranged. Both the insertable portion 151 and the non-retractable insertion support 152 are fixedly positioned relative to the base 10 and protrude from the contact surface 11. The non-retractable insertion support 152 has an insertion support tip 1523 configured for piercing the patient's skin. Fig. 8C shows an enlarged view of the non-retractable insertion support 152 and the insertable portion 151 of the sensor device with the electrons 1511,1512,1513 and the delivery needle 12, which is preferably arranged at a distance d of less than 5 mm from the insertable portion 151 measured in a plane parallel to the contact surface 11.

The patch-like medical device 1 according to this second embodiment further comprises a telescopic sleeve assembly as described above, wherein the insertable portion 151 on the insertion support 152 and the delivery needle tip 12 are both protected by the telescopic sleeve assembly 6 in its first state (Fig. 8A), and exposed when the telescopic sleeve assembly is in its second state (Fig. 8B).

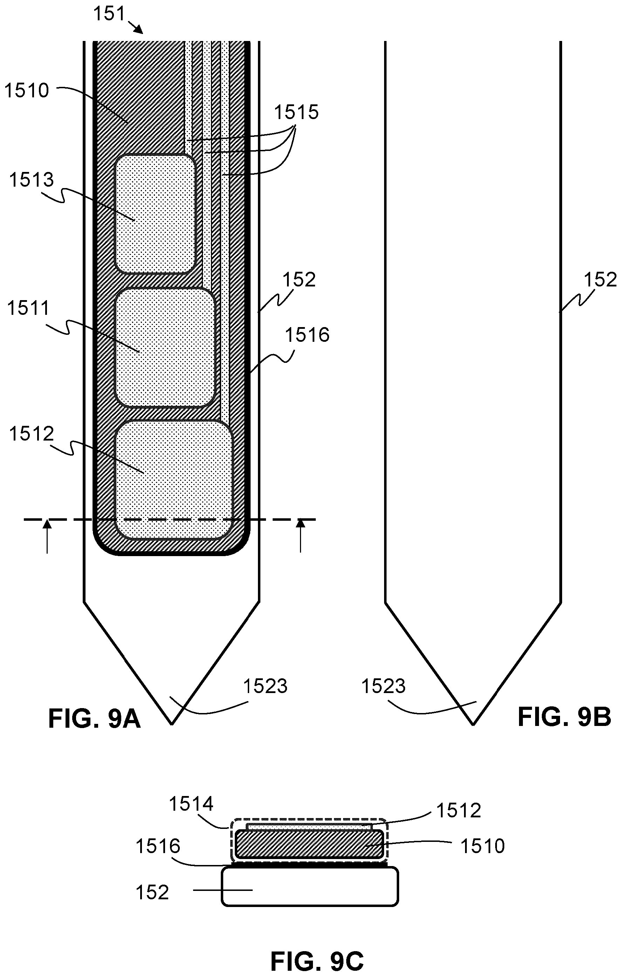

Figs. 9A to 18C show ten different examples of how the non-retractable insertion support 152 may be shaped and how the insertable portion 151 of the sensor device may be arranged on the insertion support 152. Preferably, the insertion support 152 is made of a sheet comprising or consisting of a metal, a ceramic material or any other material with a sufficient rigidity to puncture the patient's skin.

To prevent the insertable portion 151 of the sensor device from bending during insertion, the insertable portion 151 can be fixed to the insertion support 152 by fixations means, such as a fixation layer 1516 comprising biocompatible glue, an adhesive or epoxy cement, or by double-sided tape.

In each of the ten examples, the insertable portion 151 of the sensor device comprises a substrate 1510 and three electrodes which are arranged on the substrate 1510: a first electrode acting as a working electrode 1511, a second electrode acting as a reference electrode 1512 and a third electrode acting as a counter electrode 1513. The working electrode 1511, the reference electrode 1512, and the counter electrode 1513 form a three-electrode electrochemical sensor. The working electrode 1511 is the electrode where the reaction of interest occurs. The reference electrode 1512 is an electrode that is kept at a pre-determined reference electrode potential. The counter electrode 1513 is used to close the current circuit. In some embodiments (not shown in the figures), a single electrode may act as the reference electrode and the counter electrode, thus forming a two-electrode electrochemical sensor instead of a three-electrode electrochemical sensor.

The substrate 1510 is sheet-like, i.e. has an essentially rectangular cross section which is substantially wider than it is thick. The substrate can be made of a flexible material, for instance a liquid crystal polymer (LCP) or a polyimide like Kapton^{™}, which may be as thin as 50 µm.

The electrochemical sensor may be operated using electronic circuitry comprising a potentiostat for applying a well-defined potential difference between the working electrode 1511 and the reference electrode 1512 while minimizing a current through the reference electrode 1512, and for measuring a current that flows through the working electrode 1511 and/or through the counter electrode 1513. Suitable electronic circuitry is well known in the art. Conductive traces 1515 are arranged on the substrate 1510 to connect the electrodes 1511, 1512, 1513 to the electronic circuitry. In case the patch-like medical device has a first device part D comprising the insertable portion 151 of the sensor device and a separable second device part R comprising the potentiostat, the conductive traces 1515 preferably lead to contact pads (not shown in Figs. 9A to 18C), the contact pads forming a disconnectable interface between the electrodes 1511,1512,1513 and the potentiostat.

Alternatively (not shown in the figures), the electrodes 1511,1512,1513 and the conductive traces 1515 may be disposed directly onto the insertion support 152 by using printing technologies or physical/chemical vapor deposition technologies. If the insertion support 152 is made from an electrically conductive material, the insertion support 152 may be coated with a thin layer of insulating material such as parylene by chemical vapor deposition before the electrodes 1511,1512,1513 and the conductive paths 1515 are disposed onto the insertion support 152.

The insertable portion 151 may also comprise a membrane layer 1514, which at least partially envelops the electrodes 1511,1512,1513 and comprises one or more different polymers, typically 2 to 5 different polymers. The membrane may be configured to control (in particular, limit) diffusion of the analyte to the electrodes 1511,1512,1513, control (in particular, limit) diffusion of interfering substances to the electrodes 1511,1512,1513 and/or to increase biocompatibility. The membrane layer 1514 is schematically indicated in the sectional views of Figs. 9C, 10C, 11C, 12C, 13C, 14C, 15C, 16C, 17C and 18C by a dashed contour line.

In the following, the first electrode 1511 is called the working electrode, the second electrode 1512 is the called reference electrode and the third electrode 1513 is called the counter electrode. However, the roles of electrodes may be switched, i.e. each of the electrodes 1511, 1512, 1513 in Figs. 9A to 18C may be configured to act either as the working electrode, the reference electrode or the counter electrode.

### Single flat-sheet configurations

A first example of the non-retractable insertion support 152 together with the insertable portion 151 of the sensor device is shown in Figs. 9A to 9C, wherein Fig. 9A shows a front view, Fig. 9B shows a rear view, and Fig. 9C shows a sectional view along the sectional plane marked by a dashed line in Fig. 9A. In this first example, the working electrode 1511, the reference electrode 1512 and the counter electrode 1513 are arranged on a front side of the substrate 1510. The non-retractable insertion support 152 is a flat sheet with a substantially rectangular profile and has a pointed insertion support tip 1523 at its distal end to facilitate insertion into the skin. The insertable portion 151 of the sensor device is attached to the non-retractable insertion support 152 via a fixation layer 1516.

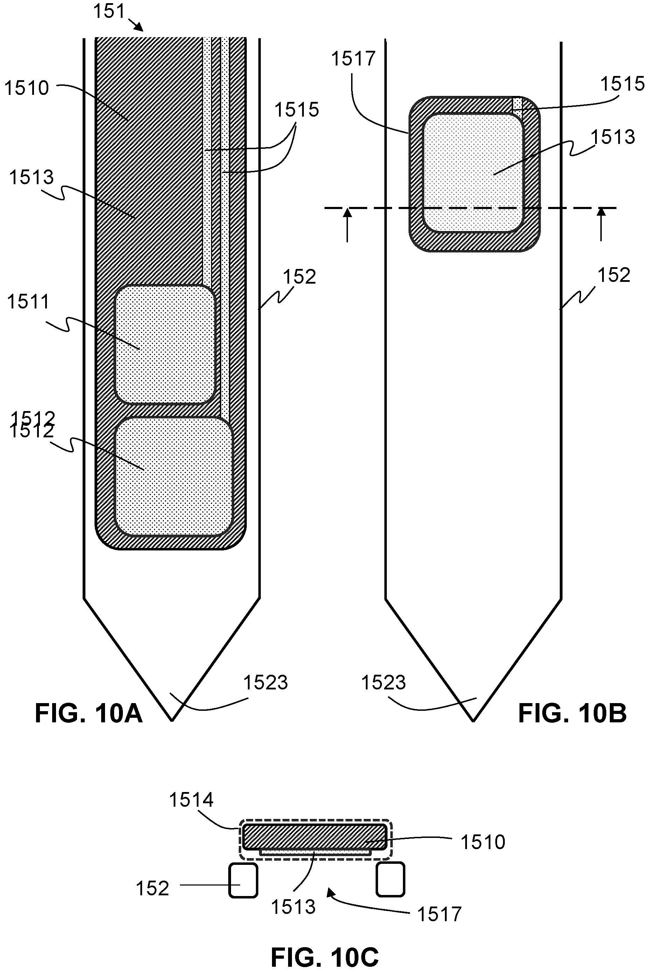

A second example of the non-retractable insertion support 152 together with the insertable portion 151 of the sensor device is shown in Figs. 10A to 10C, wherein Fig. 10A shows a front view, Fig. 10B shows a rear view, and Fig. 10C shows a sectional view along the sectional plane marked by a dashed line in Fig. 10B. In this second example, the working electrode 1511 and the reference electrode 1512 are arranged on a front side of the substrate 1510, whereas the counter electrode 1513 is arranged on a rear side of the substrate 1510. The non-retractable insertion support 152 is flat with a substantially rectangular profile and has a pointed insertion support tip 1523 at its distal end to facilitate insertion into the skin. The insertable portion 151 is arranged on a front side of the insertion support 152. The insertion support 152 exhibits a measurement window 1517 to provide access to the counter electrode 1513 from a rear side of the insertion support 152.

### "Sandwich" configurations

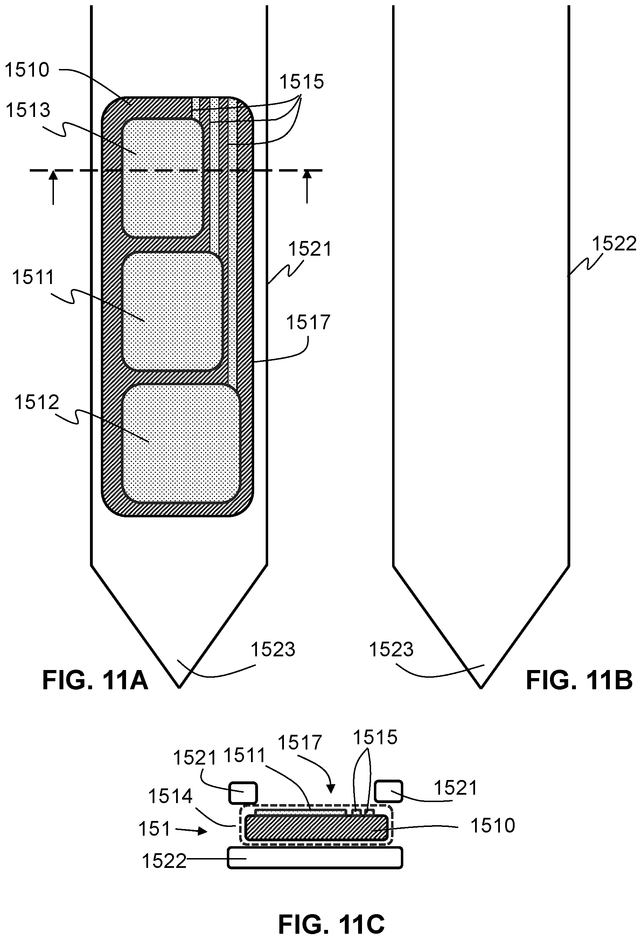

A third example of the non-retractable insertion support 152 together with the insertable portion 151 of the sensor device is shown in Figs. 11A to 11C, wherein Fig. 11A shows a front view, Fig. 11B shows a rear view, and Fig. 11C shows a sectional view along the sectional plane marked by a dashed line in Fig. 11A. In this third example, the working electrode 1511, the reference electrode 1512 and the counter electrode 1513 are arranged on a front side of the substrate 1510. The non-retractable insertion support 152 comprises a first flat sheet 1521 arranged on a front side of the substrate 1510 and a second flat sheet 1522 arranged on a rear side of the substrate 1510, such that the substrate 1510 is "sandwiched" between the first sheet 1521 and second sheet 1522. The first sheet 1521 and second sheet 1522 may be laminated to the substrate 1510 by any suitable fixation means as described above. Both the first sheet 1521 and second sheet 1522 are flat with a substantially rectangular profile and have a pointed insertion support tip 1523 at the distal end to facilitate insertion into the skin. The first sheet 1521 exhibits a measurement window 1517 to provide access to the three electrodes 1511,1512,1513 through the first flat sheet 1521.

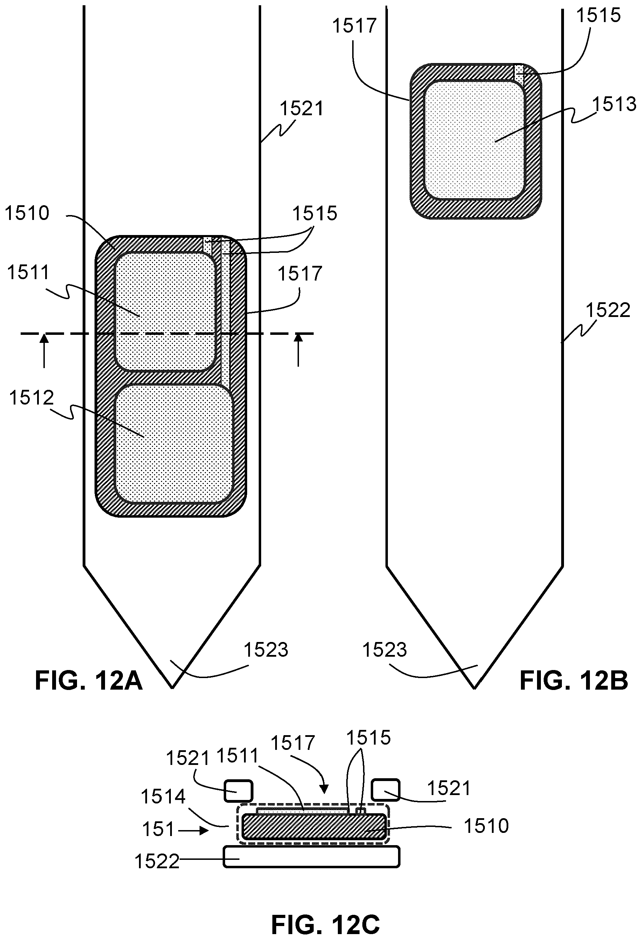

A fourth example of the non-retractable insertion support 152 together with the insertable portion 151 of the sensor device is shown in Figs. 12A to 12C, wherein Fig. 12A shows a front view, Fig. 12B shows a rear view, and Fig. 12C shows a sectional view along the sectional plane marked by a dashed line in Fig. 12A. In this fourth example, the working electrode 1511 and the reference electrode 1512 are arranged on a front side of the substrate 1510, whereas the counter electrode 1513 is arranged on a rear side of the substrate 1510. The non-retractable insertion support 152 comprises a first flat sheet 1521 arranged on a front side of the substrate 1510 and a second flat sheet 1522 arranged on a rear side of the substrate 1510, such that the substrate 1510 is "sandwiched" between the first sheet 1521 and second sheet 1522. The first sheet 1521 and second sheet 1522 may be laminated to the substrate 1510 by any suitable fixation means as described above. Both the first sheet 1521 and second sheet 1522 are flat with a substantially rectangular profile and have a pointed insertion support tip 1523 at the distal end to facilitate insertion into the skin. The first sheet 1521 exhibits a measurement window 1517 to provide access to the working electrode 1511 and the reference electrode 1512 through the first sheet 1521, whereas the second sheet 1521 exhibits a measurement window 1517 to provide access to the counter electrode 1513 through the second sheet 1522.

### "U-shaped" configurations

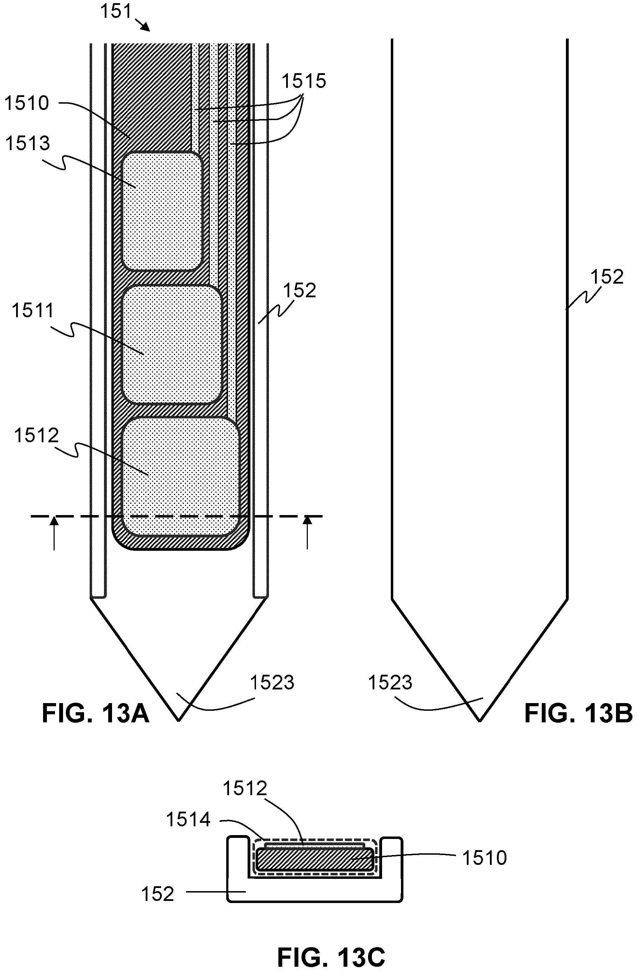

A fifth example of the non-retractable insertion support 152 together with the insertable portion 151 of the sensor device is shown in Figs. 13A to 13C, wherein Fig. 13A shows a front view, Fig. 13B shows a rear view, and Fig. 13C shows a sectional view along the sectional plane marked by a dashed line in Fig. 13A. In this fifth example, the working electrode 1511, the reference electrode 1512 and the counter electrode 1513 are arranged on a front side of the substrate 1510. The non-retractable insertion support 152 comprises a U-shaped profile covering the rear side of the substrate 1510 and providing lateral support to the substrate 1510 by having lateral portions extending from the rear side of the substrate 1510 to the front side of the substrate 1510. The lateral support may protect the substrate 1510 from being displaced sideways during insertion. The insertion support 152 and has a pointed insertion support tip 1523 at the distal end to facilitate insertion into the skin.

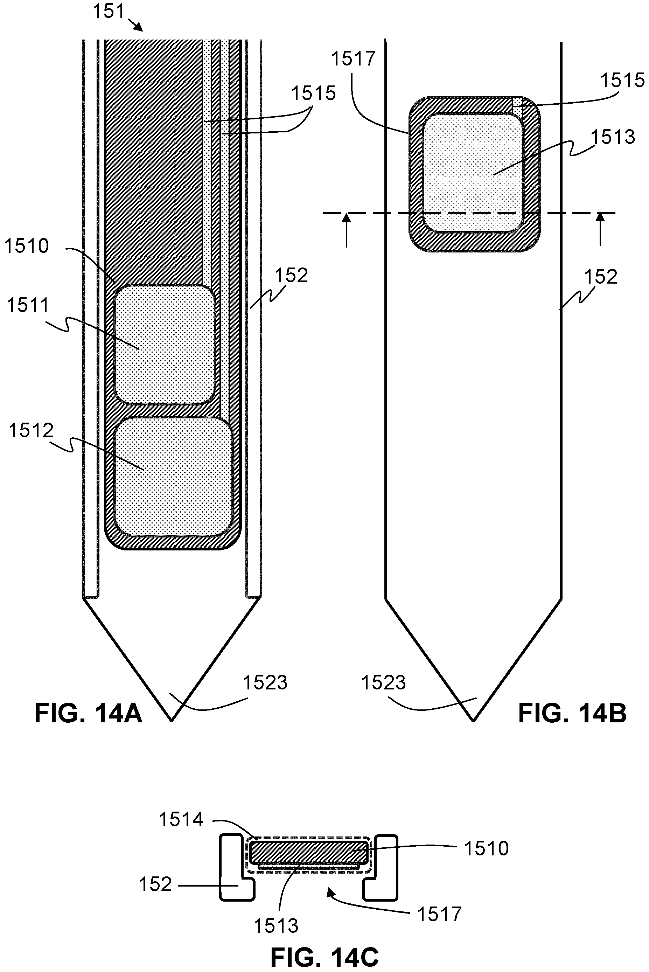

A sixth example of the non-retractable insertion support 152 together with the insertable portion 151 of the sensor device is shown in Figs. 14A to 14C, wherein Fig. 14A shows a front view, Fig. 14B shows a rear view, and Fig. 14C shows a sectional view along the sectional plane marked by a dashed line in Fig. 14B. In this sixth example, the working electrode 1511 and the reference electrode 1512 are arranged on a front side of the substrate 1510, whereas the counter electrode 1513 is arranged on a rear side of the substrate 1510. The non-retractable insertion support 152 comprises a U-shaped profile covering the rear side of the substrate 1510 and providing lateral support to the substrate 1510 by having lateral portions extending from the rear side of the substrate 1510 to the front side of the substrate 1510. The lateral support may protect the substrate 1510 from being displaced sideways during insertion. The insertion support 152 and has a pointed insertion support tip 1523 at the distal end to facilitate insertion into the skin. The insertion support 152 exhibits a measurement window 1517 to provide access to the counter electrode 1513 from a rear side of the insertion support 152.

### "C-shaped" configurations

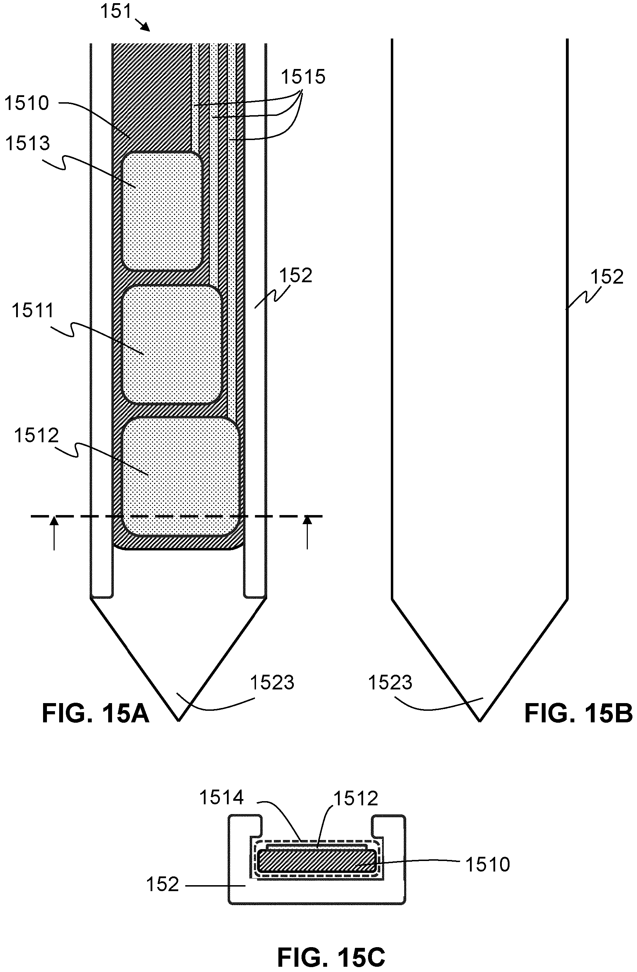

A seventh example of the non-retractable insertion support 152 together with the insertable portion 151 of the sensor device is shown in Figs. 15A to 15C, wherein Fig. 15A shows a front view, Fig. 15B shows a rear view, and Fig. 15C shows a sectional view along the sectional plane marked by a dashed line in Fig. 15A. In this seventh example, the working electrode 1511, the reference electrode 1512 and the counter electrode 1513 are arranged on a front side of the substrate 1510. The non-retractable insertion support 152 comprises a C-shaped profile covering the rear side of the substrate 1510 and providing lateral support to the substrate 1510 by having lateral portions extending from the rear side of the substrate 1510 to the front side of the substrate 1510. The lateral support may protect the substrate 1510 from being displaced sideways during insertion. In addition, the insertion support 152 has frontal portions extending partially over the front side of the substrate 1510 in parallel to the front side of the substrate 1510 to provide additional support, however without covering the electrodes 1511, 1512, 1513. The insertion support 152 and has a pointed insertion support tip 1523 at the distal end to facilitate insertion into the skin.

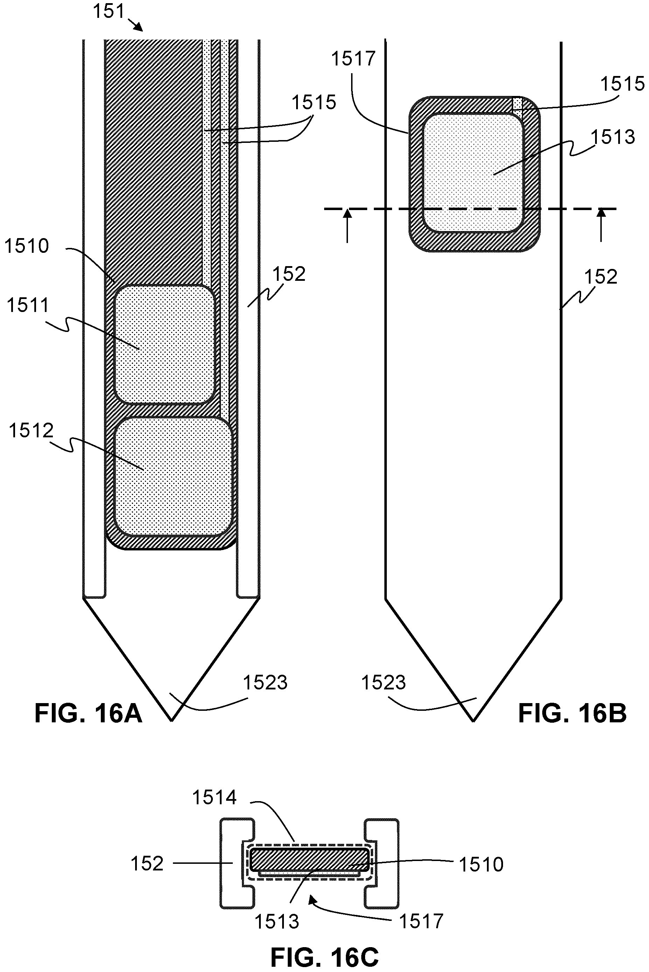

An eighth example of the non-retractable insertion support 152 together with the insertable portion 151 of the sensor device is shown Figs. 16A to 16C, wherein Fig. 16A shows a front view, Fig. 16B shows a rear view, and Fig. 16C shows a sectional view along the sectional plane marked by a dashed line in Fig. 16A. In this eighth example, the working electrode 1511 and the reference electrode 1512 are arranged on a front side of the substrate 1510, whereas the counter electrode 1513 is arranged on a rear side of the substrate 1510. The non-retractable insertion support 152 comprises a C-shaped profile covering the rear side of the substrate 1510 and providing lateral support to the substrate 1510 by having lateral portions extending from the rear side of the substrate 1510 to the front side of the substrate 1510. The lateral support may protect the substrate 1510 from being displaced sideways during insertion. In addition, the insertion support 152 has frontal portions extending partially over the front side of the substrate 1510 in parallel to the front side of the substrate 1510 to provide additional support, however without covering the electrodes 1511, 1512, 1513. The insertion support 152 and has a pointed insertion support tip 1523 at the distal end to facilitate insertion into the skin. The insertion support 152 exhibits a measurement window 1517 to provide access to the counter electrode 1513 from a rear side of the insertion support 152.

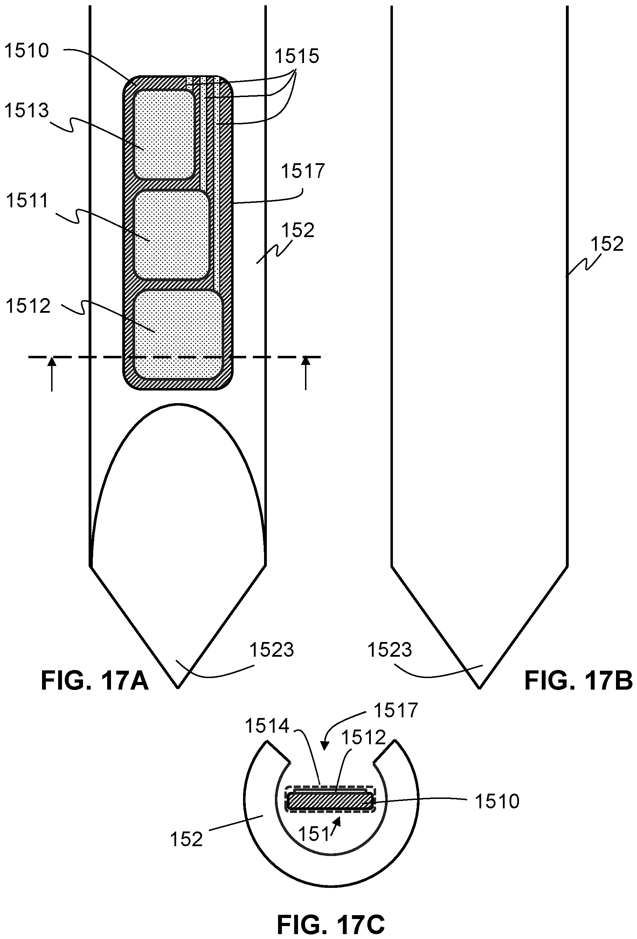

A ninth example of the non-retractable insertion support 152 together with the insertable portion 151 of the sensor device is shown in Figs. 17A to 17C, wherein Fig. 17A shows a front view, Fig. 17B shows a rear view, and Fig. 17C shows a sectional view along the sectional plane marked by a dashed line in Fig. 17B. In this ninth example, the working electrode 1511, the reference electrode 1512 and the counter electrode 1513 are arranged on a front side of the substrate 1510. The non-retractable insertion support 152 is a hollow needle with a pointed insertion support tip 1523 at the distal end to facilitate insertion into the skin. The substrate 1510 with the electrodes 1511,1512,1513 is arranged within the hollow needle. The hollow needle exhibits a measurement window 1517 to provide access to the three electrodes 1511,1512,1513.

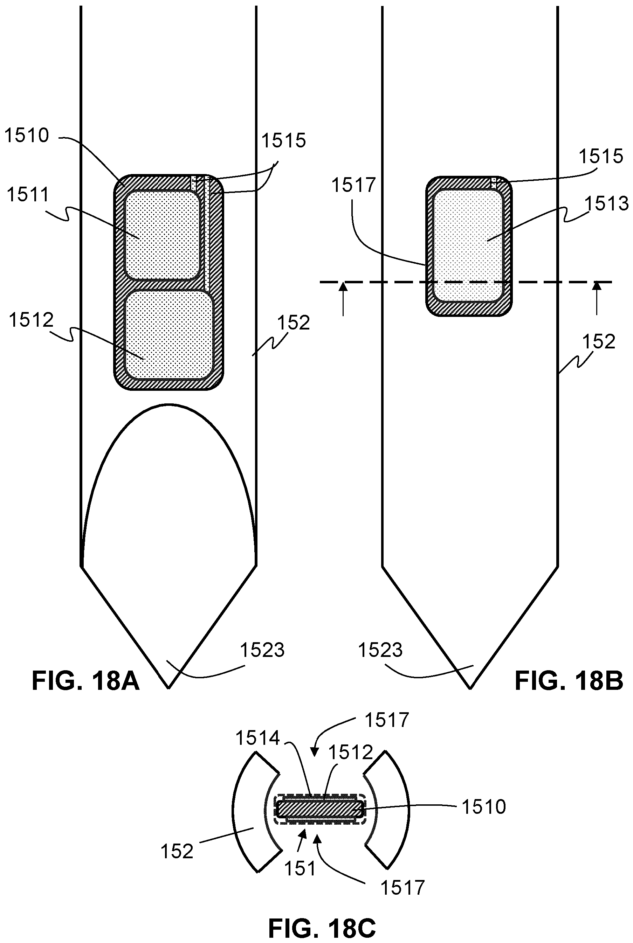

A tenth example of the non-retractable insertion support 152 together with the insertable portion 151 of the sensor device is shown in Figs. 18A to 18C, wherein Fig. 18A shows a front view, Fig. 18B shows a rear view, and Fig. 18C shows a sectional view along the sectional plane marked by a dashed line in Fig. 18B. In this tenth example, the working electrode 1511, the reference electrode 1512 and the counter electrode 1513 are arranged on a front side of the substrate 1510, whereas the counter electrode 1513 is arranged on a rear side of the substrate 1510. The non-retractable insertion support 152 is a hollow needle with a pointed insertion support tip 1523 at the distal end to facilitate insertion into the skin. The substrate 1510 with the electrodes 1511,1512,1513 is arranged within the hollow needle. The hollow needle exhibits a first measurement window 1517 to provide access to the working electrode 1511 and the reference electrode 1512 from a front side of the needle, and a second measurement window 1517 to provide access to the counter electrode 1513 from a rear side of the needle.

### LIST OF REFERENCE SIGNS

- 1: patch-like medical device
- 2: button
- 3: housing upper part
- 4: reservoir
- 5: piston
- 6: telescopic sleeve assembly
- 10: base
- 101: protrusion of the base
- 103: base wall
- 104: base wall groove
- 11: contact surface
- 12: delivery needle
- 121: delivery needle tip
- 13: recess
- 131: internal thread of the recess
- 132: top wall of the recess
- 1321: guiding bore
- 133: side wall of the recess
- 14: cylindrical element
- 15: sensor device
- 151: insertable portion of the sensor device
- 1510: substrate
- 1511: first electrode
- 1512: second electrode
- 1513: third electrode
- 1514: membrane layer
- 1515: conductive trace
- 1516: fixation layer
- 1517: measurement window
- 152: non-retractable insertion support
- 1521: first flat sheet
- 1522: second flat sheet
- 1523: insertion support tip
- 153: non-insertable portion of the sensor device
- 1531: contact portion
- 161: sharp
- 1611: sharp tip
- 1612: proximal sharp end
- 162: sharp retraction spring
- 163: switch element
- 20: protection sleeve
- 30: rotating sleeve
- 40: rotation lock sleeve
- 51: spring
- 511: first end of the spring
- 512: second end of the spring
- 60: locking mechanism
- 62: latch
- 64: clamp element
- 66: retraction-lock arm
- 661: first end of the retraction-lock arm
- 662: second end of the retraction-lock arm
- 67: push-down pins
- 68: switch element
- 681: wire loop
- 682: switch plate
- 683: switch plate guiding structures
- 684: switch strip
- 685: push button
- 686: sliding piece
- 687: resilient switch wire
- 70: fixation ring
- 823: pivot arm
- 824: first nose-like protrusion
- 8242: second nose-like protrusion
- 825: resilient wire arrangement
- 90: open retaining ring
- A: central axis
- D: first device part
- R: second device part
- L1: first assembly length
- L2: second assembly length
- d: lateral distance

## Claims

1. A patch-like medical device comprising:
a base (10) having a contact surface (11) configured to be directed towards a patient's skin;
a sensor device (15) comprising an insertable portion (151) which is configured to measure an analyte concentration subcutaneously, wherein the insertable portion (151) is fixedly positioned relative to the base (10) and protrudes from the contact surface (11);
a telescopic sleeve assembly (6) which is collapsible along a central axis (A) from a first state into a second state,
wherein in the first state, the telescopic sleeve assembly (6) has a first assembly length (L1) in direction of the central axis (A) and extends beyond the insertable portion (151), and
wherein in the second state, the telescopic sleeve assembly (6) has a second assembly length (L2) in direction of the central axis (A) that is shorter than the first assembly length (L1) and the insertable portion (151) protrudes from the telescopic sleeve assembly (6).

2. The patch-like medical device of claim 1, wherein in the second state, the telescopic sleeve assembly (6) is arranged inside the patch-like medical device (1) and does not protrude from the contact surface (11).

3. The patch-like medical device of claim 1 or 2, further comprising:
a sensor insertion mechanism comprising a sharp (161) having a sharp tip (1611) configured for piercing the patient's skin, the sharp being movable from an insertion position to a retracted position,
wherein in the insertion position, the insertable portion (151) of the sensor device (15) is at least partially received within the sharp (161) and the sharp tip (161) extends beyond the insertable portion (151) of the sensor device (15) to enable insertion of the insertable portion (151) into the patient's skin, and
wherein in the retracted position, the insertable portion (151) of the sensor device (15) is exposed.

4. The patch-like medical device of claim 3, wherein the sensor insertion mechanism comprises a retraction drive mechanism configured to drive the movement of the sharp (161) from the insertion position to the retracted position.

5. The patch-like medical device of claim 4, wherein the retraction drive mechanism comprises a sharp retraction spring (162) for driving the movement of the sharp (161),
wherein in the insertion position, and sharp retraction spring (162) is in a biased state.

6. The patch-like medical device of any one of claims 3 to 5, wherein the sensor insertion mechanism further comprises:
a retraction-lock mechanism having
a retraction-lock state, in which the sharp (161) is held in the insertion position, and
a released state, in which the sharp (161) is allowed to move to the retracted state, and
wherein the sensor insertion mechanism further comprises:
a retraction trigger mechanism configured to switch the retraction-lock from the retraction-lock state to the released state.

7. The patch-like medical device of claim 6,
wherein the retraction trigger mechanism comprises a switch element (68) to switch the retraction-lock mechanism from the retraction-lock state to the released state, the switch element (68) comprising or consisting of a shape-memory alloy.

8. The patch-like medical device of claim 6, further comprising:
a locking mechanism (60) having
a locked state in which the locking mechanism (60) prevents the telescopic sleeve assembly (6) from collapsing into the second state when the telescopic sleeve assembly (6) is in the first state, and
an unlocked state in which the locking mechanism (60) allows the telescopic sleeve assembly (6) to collapse into the second state, and
a trigger mechanism configured to switch the locking mechanism from the locked state to the unlocked state;
wherein the trigger mechanism for switching the locking mechanism from the locked state to the unlocked state and the retraction trigger mechanism for switching the retraction-lock mechanism from the retraction-lock state to the released state share a movable component (823).

9. The patch-like medical device of claim 8, wherein the movable component (823) is movable from a sleeve-unlocking position to a retraction-releasing position,
wherein in the sleeve-unlocking position, the locking mechanism is allowed to move to the unlocked state and thereby cause the telescopic sleeve assembly to collapse into the second state, but the retraction-lock mechanism is kept in the retraction-lock state, in which the sharp is held in the insertion position, and
wherein in the retraction-releasing position, the retraction-lock mechanism is allowed to move to the released state and thereby cause the sharp (161) to move to the retracted state.

10. The patch-like medical device of claim 1 or 2, wherein the sensor device (15) comprises a non-retractable insertion support (152) on which the insertable portion (151) is arranged, the insertion support (152) having an insertion support tip (1523) configured for piercing the patient's skin.

11. The patch-like medical device of any one of the preceding claims, further comprising:
a delivery needle (12) fixedly positioned relative to the base (10), the delivery needle (12) having a delivery needle tip (121) which protrudes from the contact surface (11) and which is configured for piercing the patient's skin,
wherein the telescopic sleeve assembly (6) extends beyond the delivery needle tip (121) to prevent the delivery needle tip (121) from piercing the patient's skin in the first state, and exposes the delivery needle tip (121) to enable piercing the patient's skin in the second state.

12. The patch-like medical device of claim 11, wherein the insertable portion (151) and the delivery needle tip (121) are spaced apart by a lateral distance (d) of less than 5 mm, the lateral distance (d) being defined in a plane parallel to the contact surface (11).

13. A patch-like medical device comprising:
a base (10) having a contact surface (11) configured to be directed towards a patient's skin;
a sensor device (12) comprising an insertable portion (151) which is configured to measure an analyte concentration subcutaneously, wherein the insertable portion (151) is fixedly positioned relative to the base (10) and protrudes from the contact surface (11);
a sensor insertion mechanism comprising:
a sharp (161) having a sharp tip (1611) configured for piercing the patient's skin, the sharp (161) being movable from an insertion position to a retracted position, and
a retraction drive mechanism configured to drive the movement of the sharp (161) from the insertion position to the retracted position,
wherein in the insertion position, the insertable portion (151) of the sensor device (15) is at least partially received within the sharp (161) and the sharp tip (1611) extends beyond the insertable portion (151) of the sensor device (15) to enable insertion of the insertable portion (151) into the patient's skin, and
wherein in the retracted position, the insertable portion (151) of the sensor device is exposed.

14. The patch-like medical device of claim 13, wherein the retraction drive mechanism comprises a sharp retraction spring (162) for driving the movement of the sharp (161),
wherein in the insertion position, the sharp retraction spring (162) is in a biased state.

15. The patch-like medical device of claim 13 or 14, wherein the sensor insertion mechanism further comprises:
a retraction-lock mechanism having
a retraction-lock state, in which the sharp (161) is held in the insertion position, and
a released state, in which the sharp (161) is allowed to move to the retracted state, and
wherein the sensor insertion mechanism further comprises:
a retraction trigger mechanism configured to switch the retraction-lock from the retraction-lock state to the released state.

16. The patch-like medical device of claim 15, wherein the retraction-lock mechanism comprises a switch element (68) to switch the retraction-lock from the retraction-lock state to the released state, the switch element (68) comprising or consisting of a shape-memory alloy.

17. A patch-like medical device comprising:
a base (10) having a contact surface (11) configured to be directed towards a patient's skin;
a sensor device (12) comprising:
an insertable portion (151) which is configured to measure an analyte concentration subcutaneously, and
a non-retractable insertion support (152) on which the insertable portion (151) is arranged,
wherein the insertable portion (151) and the non-retractable insertion support (152) are fixedly positioned relative to the base (10) and protrude from the contact surface (11), and
wherein the non-retractable insertion support (152) has an insertion support tip (1523) configured for piercing the patient's skin, and
a delivery needle (12) fixedly positioned relative to the base (10), the delivery needle (12) having a delivery needle tip (121) which protrudes from the contact surface (11) and which is configured for piercing the patient's skin.

18. The patch-like medical device of claim 17, wherein the insertable portion (151) and the delivery needle tip (121) are spaced apart by a lateral distance of less than 5 mm, the lateral distance being defined in a plane parallel to the contact surface (11),
the insertion support tip (1611) and the delivery needle tip (121) preferably extending from the contact surface (11) in parallel and forming an angle of 90° with the contact surface (11).
